(19) Europäisches Patentamt
European Patent Office
Office européen des brevets

(11) **EP 4 251 763 B1**

(12) **EUROPEAN PATENT SPECIFICATION**

(45) Date of publication and mention
of the grant of the patent:
**25.12.2024  Bulletin 2024/52**

(21) Application number: **21816141.2**

(22) Date of filing: **24.11.2021**

(51) International Patent Classification (IPC):
***C12Q 1/6806*** *(2018.01)*    ***C12Q 1/6848*** *(2018.01)*

(52) Cooperative Patent Classification (CPC):
(C-Sets available)
**C12Q 1/6806; C12Q 1/6848**          (Cont.)

(86) International application number:
**PCT/GB2021/053036**

(87) International publication number:
**WO 2022/112751 (02.06.2022 Gazette 2022/22)**

(54) **METHODS FOR THE ACCURATE DETECTION OF MUTATIONS IN SINGLE MOLECULES OF DNA**

VERFAHREN ZUR GENAUEN DETEKTION VON MUTATIONEN IN EINZELNEN DNA-MOLEKÜLEN

PROCÉDÉS POUR LA DÉTECTION PRÉCISE DE MUTATIONS DANS DES MOLÉCULES UNIQUES D'ADN

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority:  **24.11.2020  GB 202018476**

(43) Date of publication of application:
**04.10.2023  Bulletin 2023/40**

(73) Proprietor: **Genome Research Limited
Cambridge, Cambridgeshire CB10 1SA (GB)**

(72) Inventors:
 • **ABASCAL, Federico**
**Hinxton,Cambridge CB10 1SA (GB)**
 • **LENSING, Stefanie**
**Hinxton,Cambridge CB10 1SA (GB)**
 • **MARTINCORENA, Inigo**
**Hinxton,Cambridge CB10 1SA (GB)**
 • **OSBORNE, Robert**
**Hinxton,Cambridge CB10 1SA (GB)**

(74) Representative: **Sagittarius IP
Marlow International
Parkway
Marlow SL7 1YL (GB)**

(56) References cited:
**EP-A1- 3 385 379        WO-A1-2017/218979**

WO-A1-2018/045141    WO-A1-2018/053070
WO-A1-2019/126803    WO-A1-2021/022046
WO-A2-2007/087310    CN-A- 111 893 170
US-A1- 2016 215 331

 • **YOU XINYUE ET AL: "Detection of genome-wide low-frequency mutations with Paired-End and Complementary Consensus Sequencing (PECC-Seq) revealed end-repair-derived artifacts as residual errors", ARCHIVES OF TOXICOLOGY, SPRINGER, DE, vol. 94, no. 10, 31 July 2020 (2020-07-31), pages 3475 - 3485, XP037249868, ISSN: 0340-5761, [retrieved on 20200731], DOI: 10.1007/S00204-020-02832-0**
 • **ABASCAL FEDERICO ET AL: "Somatic mutation landscapes at single-molecule resolution", NATURE,, vol. 593, no. 7859, 28 April 2021 (2021-04-28), pages 405 - 410, XP037456141, [retrieved on 20210428], DOI: 10.1038/S41586-021-03477-4**
 • **MARGARET L. HOANG ET AL: "Genome-wide quantification of rare somatic mutations in normal human tissues using massively parallel sequencing", PROCEEDINGS OF THE NATIONAL ACADEMY OF SCIENCES, vol. 113, no. 35, 15 August 2016 (2016-08-15), pages 9846 - 9851, XP055393458, ISSN: 0027-8424, DOI: 10.1073/pnas.1607794113**

(52)  Cooperative Patent Classification (CPC): (Cont.)

C-Sets
**C12Q 1/6806, C12Q 2521/301, C12Q 2525/173,
C12Q 2525/186, C12Q 2525/191, C12Q 2535/122,
C12Q 2563/179;
C12Q 1/6848, C12Q 2521/301, C12Q 2525/173,
C12Q 2525/186, C12Q 2525/191, C12Q 2535/122,
C12Q 2563/179**

**Description**

**FIELD OF THE INVENTION**

The invention relates to a method of generating a nucleic acid library for sequencing with error rates <1 error per 100 million base pairs and uses thereof. Provided are methods for detecting mutations (including driver mutations) present in single DNA molecules, such as a single DNA molecule from a polyclonal population of cells, and use of the methods in measuring somatic mutation rates, mutational signatures and driver mutations *in vivo* or *in vitro.* Also provided are methods of discovering somatic mutations involved in disease and detecting mutations from cells in culture, diagnosing disease and for detecting the presence and/or identity of a suspected mutation in a cell cultured *in vitro* and/or subjected to a mutagenic process *in vitro.* Further provided is a method of computational analysis of duplex sequencing data.

**BACKGROUND OF THE INVENTION**

Adult tissues are typically polyclonal, composed of genetically heterogeneous cells or clones of cells carrying different somatic mutations accumulated throughout life. Studying somatic mutagenesis in normal tissues has required novel approaches to circumvent this genetic heterogeneity, such as ultra-deep sequencing of small biopsies to detect small clones (Martincorena *et al.* (2015) doi: https://doi.org/10.1126/science.aaa6806 and Martincorena *et al.* (2018) doi: https://doi.org/10.1126/science.aau3879), laser microdissection of microscopic histological units (Lee-Six *et al.* (2019) doi: https://doi.org/10.1038/s41586-019-1672-7 and Brunner *et al.* (2019) doi: https://doi.org/10.1038/s41586-019-1670-9), isolation of single-cells followed by *in vitro* expansion into clonal organoids or colonies (Welch *et al.* (2012) doi: https://doi.org/10.1016/j.cell.2012.06.023, Jager *et al.* (2017) doi: https://doi.org/10.1038/ nprot.2017.111 and Franco *et al.* (2018) doi: https://doi.org/10.1038/s41467-018-03244-6), or direct single-cell sequencing using whole-genome amplification (Lodato *et al.* (2015) doi: http://doi.org/10.1126/science.aab1785 and Lodato *et al.* (2018) doi: https://doi.org/10.1126/ science.aao4426). While these approaches are transforming our understanding of the mutational landscape of several tissues, the error rate of single-cell sequencing is often high due to amplification errors, and all other approaches are generally limited to mitotically-active cell types that are either arranged into clonal patches within tissues or that can be expanded *in vitro* from single cells. As a result, the rates and patterns of somatic mutation across most human cell types remain unknown.

The fundamental limitation of standard sequencing approaches for the study of genetically heterogenous samples is the need to detect the same mutation in multiple cells to distinguish genuine mutations from sequencing errors, a consequence of the error rate of these technologies. While standard sequencing technologies typically have error rates above $1\times10^{-3}$ errors/bp, most somatic tissues sequenced to date have mutation loads on the order of $1\times10^{-8}$ to $1\times10^{-6}$ mutations/bp (Abascal *et al.* (2021) doi: https://doi.org/10.1038/ s41586-021-03477-4; Moore *et al.* (2021) doi: https://doi.org/10.1038/s41586-021-03822-7; and Cagan *et al.* (2021) doi: https://doi.org/10.1101/2021.08.19.456982). Several protocols have been developed to increase the accuracy of standard sequencing methods by tagging individual molecules of DNA with unique molecular barcodes and reading the same molecule multiple times, reducing error rates by single-molecule consensus sequencing (Hiatt *et al.* (2010) doi: https://doi.org/10.1038/ nmeth.1416, Casbon *et al.* (2011) doi: https://doi.org/ 10.1093/nar/gkr217, Kinde *et al.* (2011) doi: https://doi.org/10.1073/pnas.1105422108). The most accurate approaches use duplex consensus sequencing (Schmitt *et al.* (2012) doi: https://doi.org/10.1073/pnas.1208715109), sequencing copies of both strands of a DNA molecule to remove sequencing errors present in individual reads and PCR errors present in copies of one of the two strands. However, mapping errors - particularly in repetitive regions of the genome - and the accidental copying of errors between strands during library preparation violate the independence of both strands and limit the accuracy of such approaches (Abascal *et al.* (2021)). The error rate of standard duplex sequencing using sonication and end repair is reported to be higher than $10^{-7}$ errors/bp (Abascal *et al.* (2021)).

WO 2019/126803 discloses methods for preparing sequencing libraries from a DNA-containing sample, as well as methods for reducing edge errors resulting from the deamination of 5' end overhanging cytosine residues to uracil.

US 2016/215331 discloses methods of genotyping by sequencing, comprising blunt end digestion of genomic DNA and ligation of sequencing adaptors, followed by size-selection to capture a small portion of the genome that is consistent between samples.

WO 2007/087310 discloses the tagging of nucleic acid fragments with unique sequence tokens, as well as the use of said tokens in determining sequence variations in the associated nucleic acid fragments.

There is therefore a need for methods with higher accuracy, such as methods which generate sequence data with less than $10^{-7}$ errors/bp, to enable the accurate detection and identification of somatic mutations from cell populations.

## SUMMARY OF THE INVENTION

According to a first aspect of the invention, there is provided a method of generating a nucleic acid library for sequencing, said method comprising the steps of:

(i) fragmenting a nucleic acid composition to generate fragmented nucleic acid molecules with blunt ends;
(ii) introducing dideoxy nucleotides at internal nick sites present in the fragmented nucleic acid molecules with blunt ends and tailing to generate tailed nucleic acid fragments; and
(iii) adding sequencing adaptors to the tailed nucleic acid fragments to generate a nucleic acid library.

In some embodiments, fragmenting the nucleic acid composition in step (i) comprises using one or more blunt/non-cohesive end-generating endonuclease. In other embodiments, fragmenting the nucleic acid composition comprises mechanical fragmentation, such as sonication, and removing overhangs from the fragmented nucleic acid molecules using one or more exonuclease enzyme, such as Mung Bean nuclease, to generate fragmented nucleic acid molecules with blunt ends.

Thus, according to another aspect of the invention there is provided a method for generating a nucleic acid library for sequencing, said method comprising the steps of:

(i a) fragmenting a nucleic acid composition by sonication to generate fragmented nucleic acid molecules;
(i b) removing overhangs from the fragmented nucleic acid molecules using one or more exonuclease enzyme to generate fragmented nucleic acid molecules with blunt ends;
(ii) introducing dideoxy nucleotides at internal nick sites present in the fragmented nucleic acid molecules and tailing to generate tailed nucleic acid fragments; and
(iii) adding sequencing adaptors to the tailed nucleic acid fragments to generate a nucleic acid library.

In a further embodiment, the method additionally comprises the step of:
(iv) selectively enriching nucleic acids corresponding to one or more genomic region of interest to generate an enriched nucleic acid library.

According to a further aspect of the invention, there is provided a method for detecting mutations present in single DNA molecules, said method comprising the steps of:

(i) generating a nucleic acid library or an enriched nucleic acid library using the method described herein;
(ii) sequencing the nucleic acid library or the enriched nucleic acid library to generate sequencing data; and
(iii) computational analysis of the sequencing data of step (ii) to detect the presence and/or identity of a mutation in single DNA molecules.

In a further aspect, there is provided a method for detecting a mutation present in a single DNA molecule from a population of cells, comprising performing the method described herein.

In a yet further aspect, there is provided a method of diagnosing disease in a human or animal subject, said method comprising the steps of:

(i) detecting the presence and/or identity of a mutation in a single DNA molecule according to the method described herein; and
(ii) using the presence and/or identity of the mutation as an indicator of disease in the human or animal subject.

In a still further aspect, there is provided an *in vitro* method for detecting the presence and/or identity of a suspected mutation in a cell, said method comprising the steps of:

(i) culturing a cell *in vitro* and/or subjecting an *in vitro* cell culture to a mutagenic process, such as by performing gene editing; and
(ii) detecting the presence and/or identity of a mutation in a single DNA molecule in the cell or cell culture, according to the method described herein.

## BRIEF DESCRIPTION OF THE FIGURES

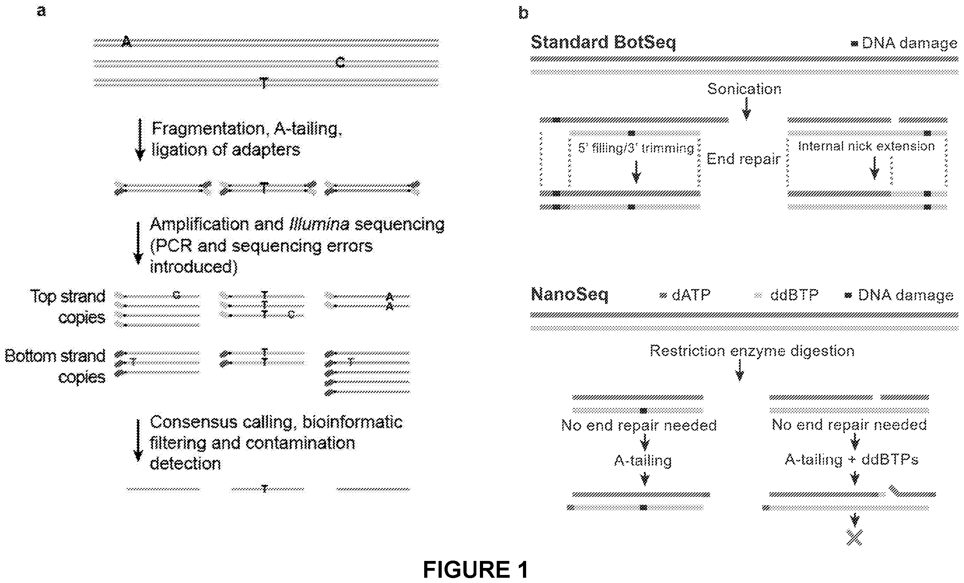

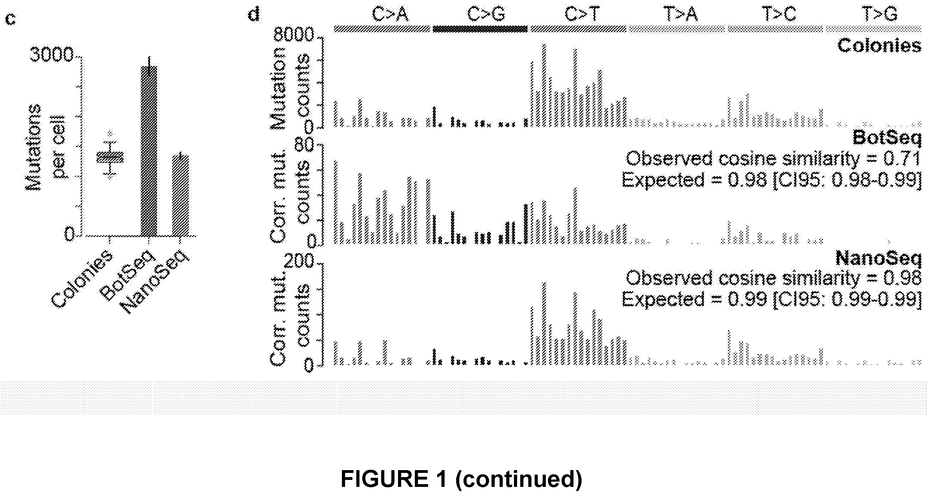

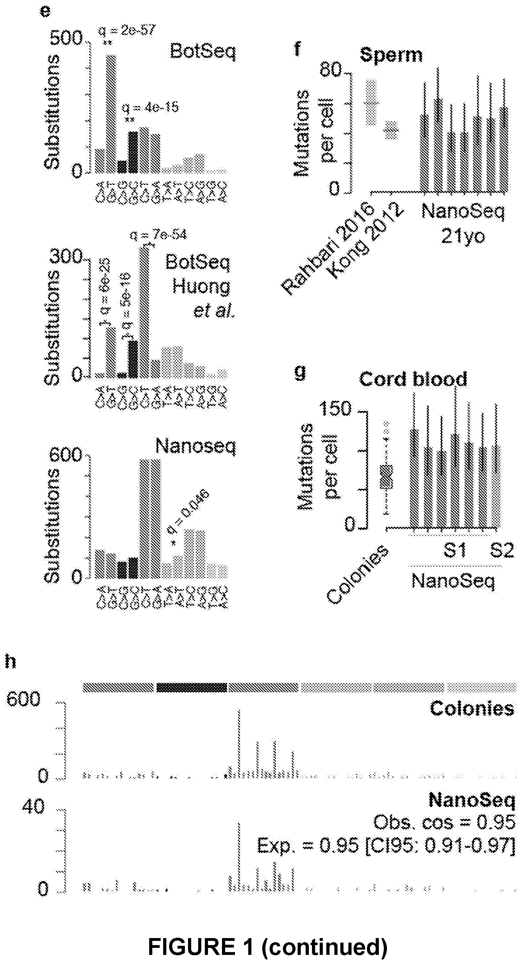

**Figure 1:** Standard BotSeqS and NanoSeq sequencing protocols. **A)** Fundamentals of duplex sequencing protocols.

**B)** Standard BotSeqS (top) and the new NanoSeq approach (bottom) for genome fragmentation and library preparation. **C)** Mutation burden estimates in granulocytes using BotSeqS and NanoSeq, compared to standard results with single-cell derived blood colonies. Boxplot notches show the 95% confidence interval for the median. BotSeqS and NanoSeq bars show 95% Poisson confidence intervals. **D)** Comparison of BotSeqS and NanoSeq granulocytes substitution profiles with blood colonies data. **E)** High substitution imbalances are present in standard BotSeqS protocols but absent from NanoSeq. Imbalances were tested with a binomial test assuming p of 0.5 and p-values were corrected with Benjamini and Hochberg's FDR method. **F)** NanoSeq mutation burden estimates for seven sperm samples from a 21-year-old donor, compared to reported estimates of mutation burden in sperm, showing 95% Poisson confidence intervals. **G)** NanoSeq mutation burden estimates for cord blood granulocytes and how these compare to single-cell derived cord blood colonies, showing 95% Poisson confidence intervals; Boxplot notches show the 95% confidence interval for the median, with the mean and its 95% confidence interval shown inside. **H)** Comparison between cord blood colonies and granulocytes substitution profiles.

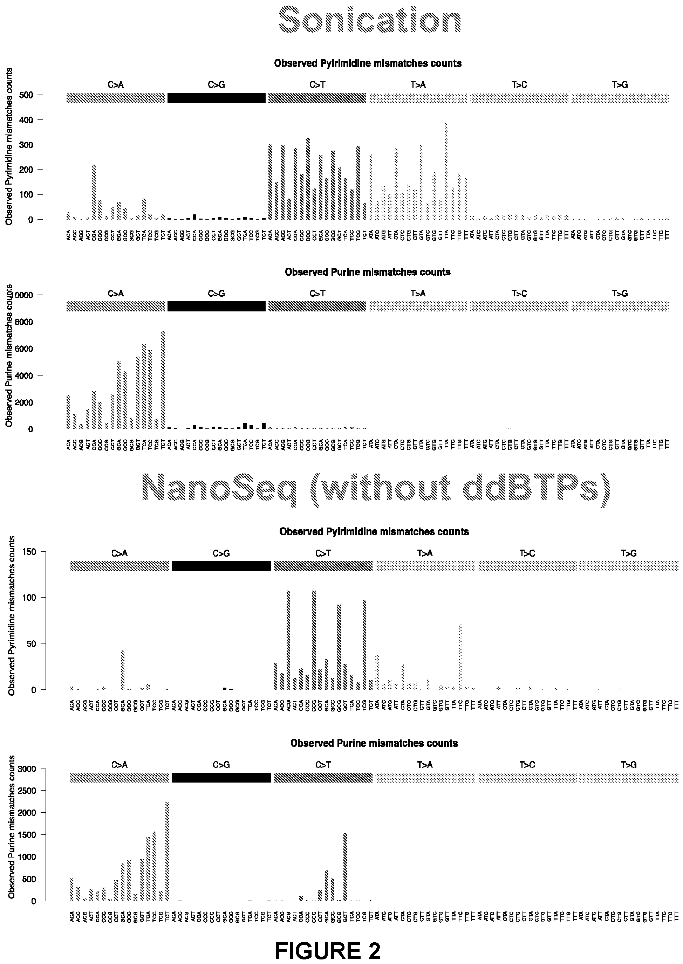

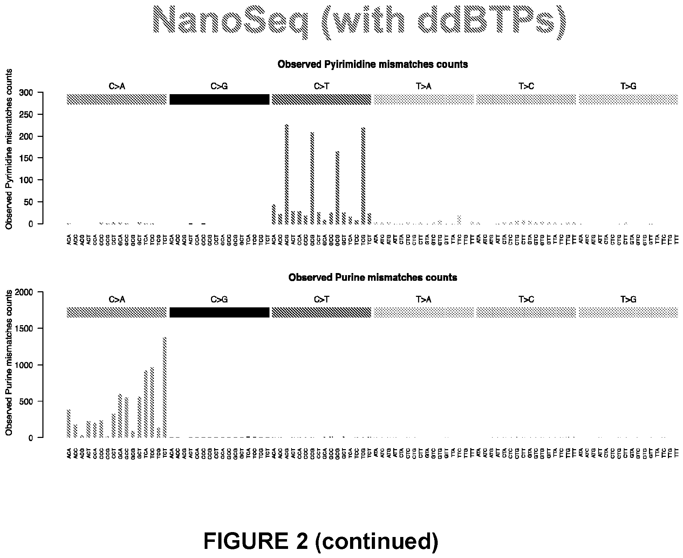

**Figure 2:** Single-strand consensus for pyrimidine (top) and purine (bottom) substitutions. For example, C>T are shown on top, while complementary G>A are shown on bottom. **A)** Single-strand consensus profile for sonication. **B)** and **C)** Single-strand consensus profiles for the standard and modified A-tailing protocols, respectively. By including ddBTPs C>A, G>A and T>A calls are largely reduced, hence reducing the risk of false positive double-strand consensus calls.

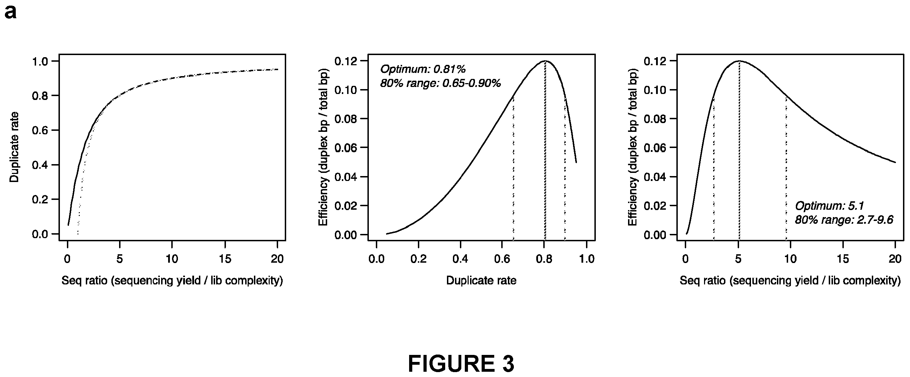

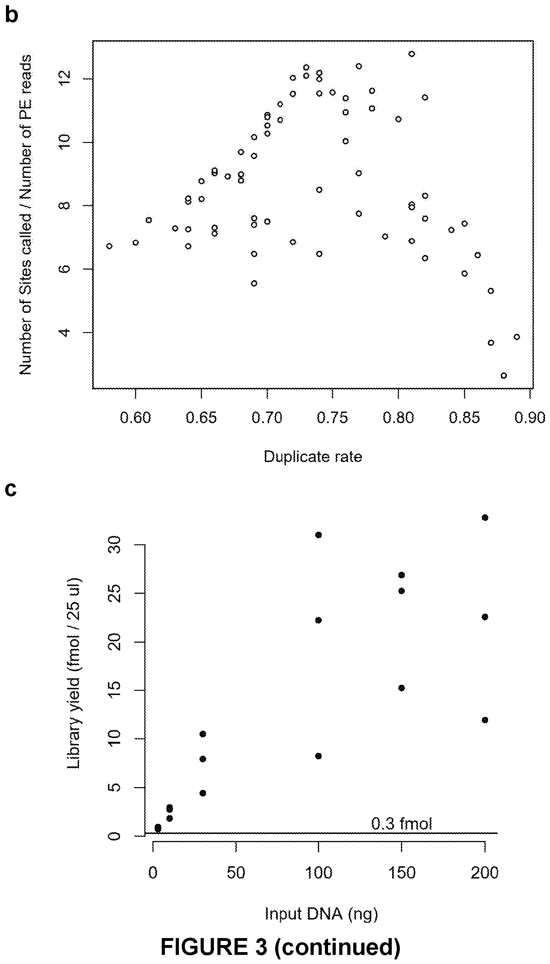

**Figure 3: A)** Analytical estimation of the relationship between sequencing yield, library complexity, duplicate rates and efficiency. From left to right: duplicate rate as a function of the sequencing ratio (sequencing yield / library complexity); efficiency (measured as bases called/ bases sequenced) as a function of the duplicate; and efficiency as a function of sequencing ratio. **B)** Empirical relationship between duplicate rates and efficiency of the method, measured as bases called / number of paired-end reads sequenced. **C)** NanoSeq protocol library yields (fmol in 25μl) for different amounts of input DNA.

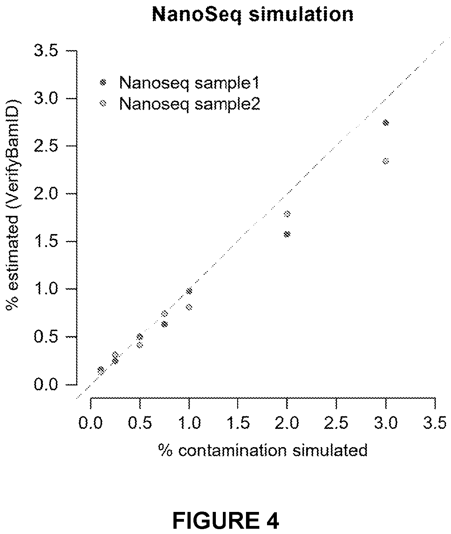

**Figure 4:** Contamination simulations using two NanoSeq samples mixed together. The % of contamination estimated is the prediction made by VerifyBamID.

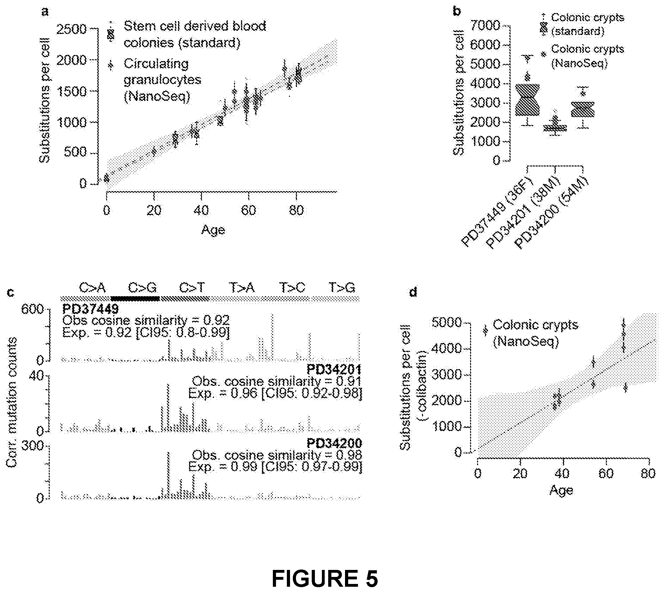

**Figure 5:** Mutation analysis of differentiated cells. **A)** Substitution per cell estimates for donors of different ages, comparing NanoSeq on granulocytes to standard sequencing of single cell-derived blood colonies (dark boxplots); boxplots and confidence intervals are displayed as in **Figure 1C;** dashed lines are linear mixed regression models; linear mixed model 95% confidence intervals for NanoSeq data calculated through parametric bootstrapping. For granulocytes, the intercept is 137.6 [$CI_{95\%}$: -117.6-413.2] and the slope 20.6 [15.8-25.2]. For blood colonies, the intercept is 120.4 [27.9-218.5] and the slope 19.8 [18.2-21.4]. **B)** Comparison between standard methods and NanoSeq burden estimates for colonic crypts from three donors. **C)** Substitution profiles for colonic crypts from the three donors and cosine similarities to profiles obtained with standard methods. **D)** Accumulation of substitutions throughout life in colonic crypts from 5 donors, excluding substitutions attributed to the episodic colibactin signature; confidence intervals as in panel **A.** Intercept of 156.9 [-1776.8-2117.7] and slope of 50.9 [9.8-91.1].

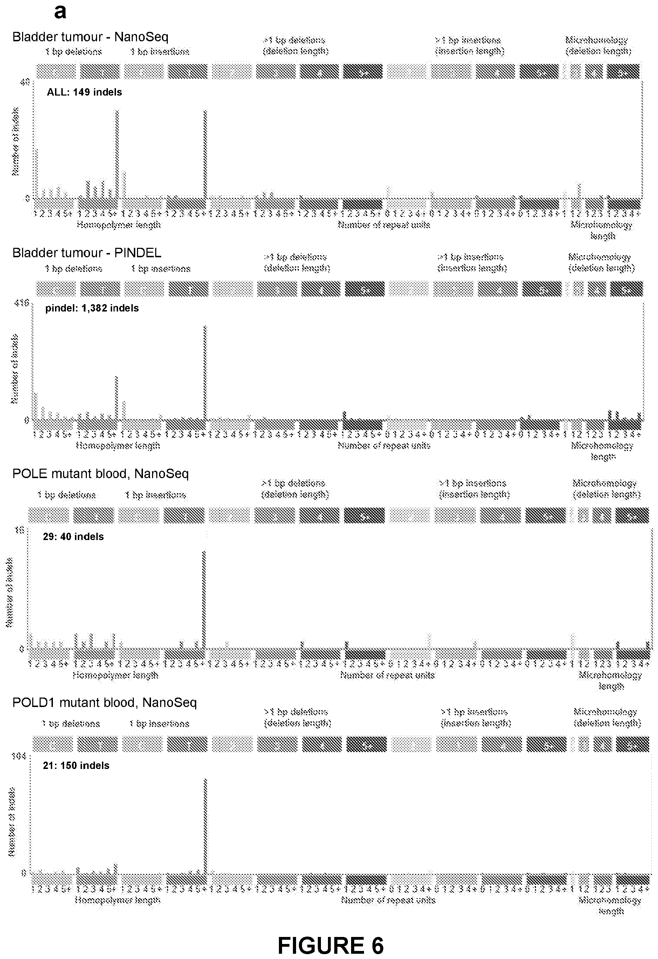

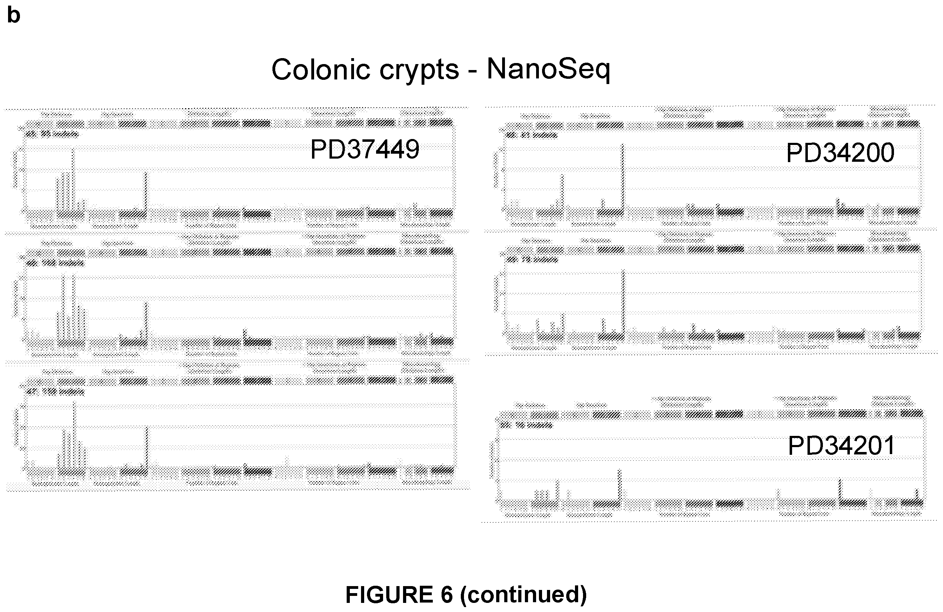

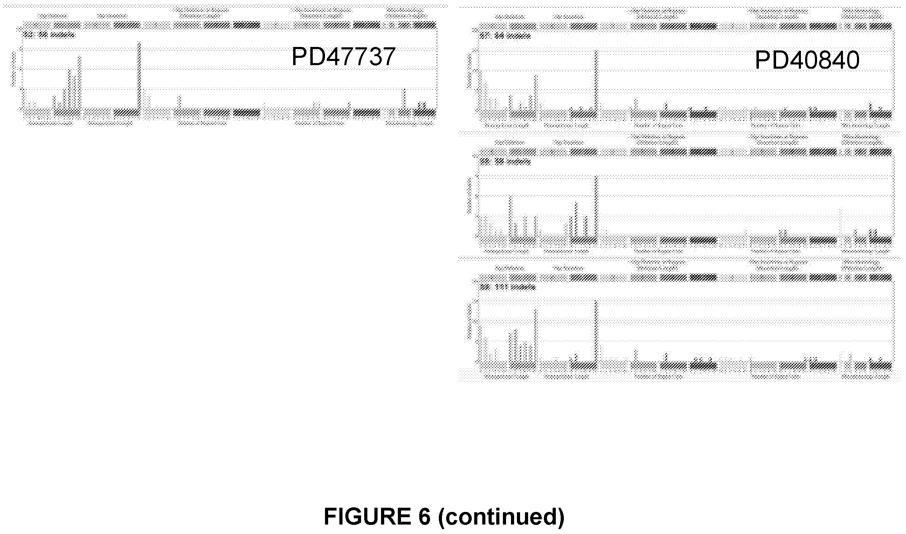

**Figure 6: A)** Comparison between the indel profiles obtained based on NanoSeq and standard data for a bladder tumour. NanoSeq indels were called as described herein; standard data indels were called with Pindel. **B)** Indel profiles for all the colonic crypts analysed, showing the colibactin signature in PD37449 (left column) and indels in POLE and POLD1 mutants (right column).

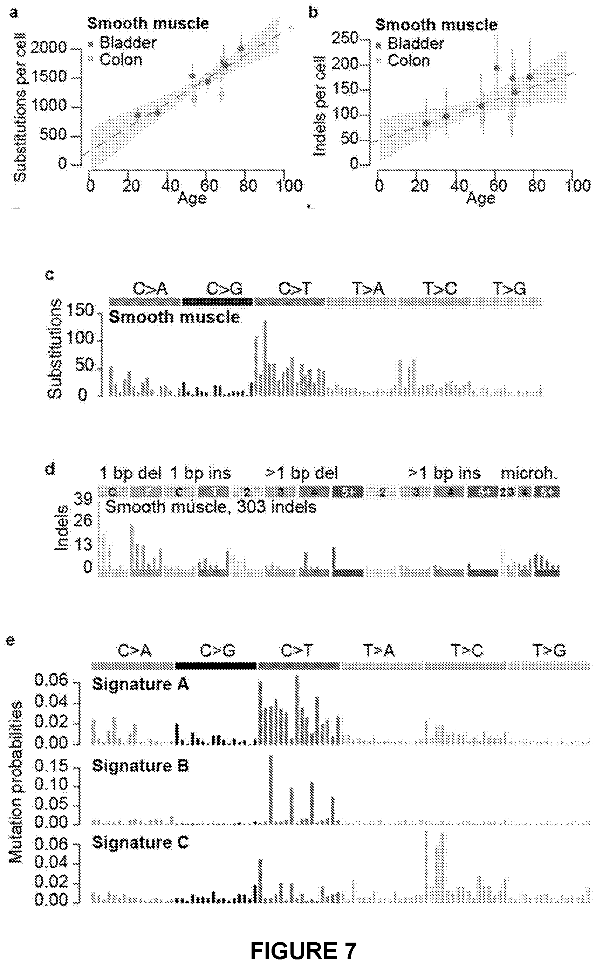

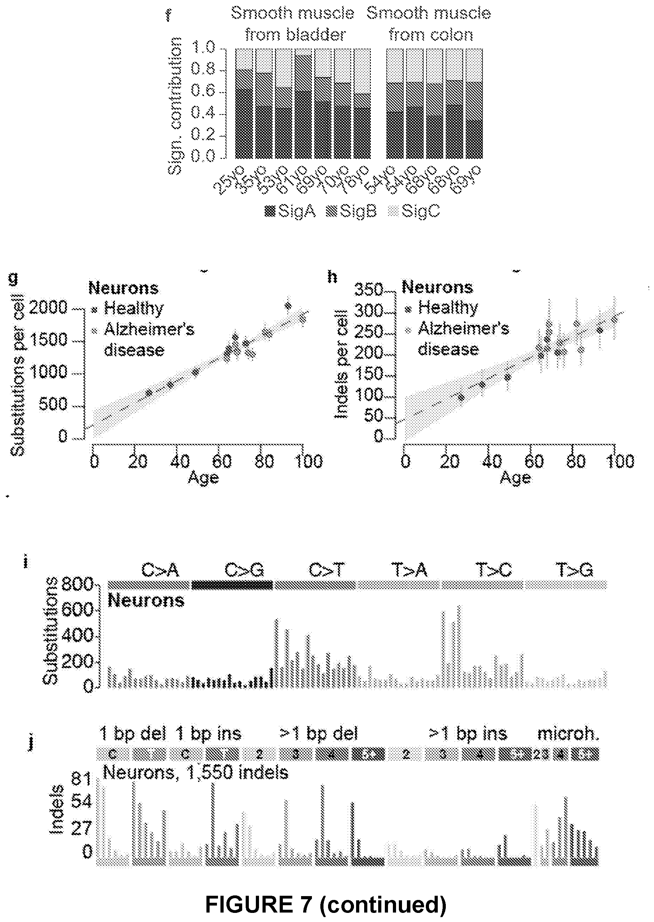

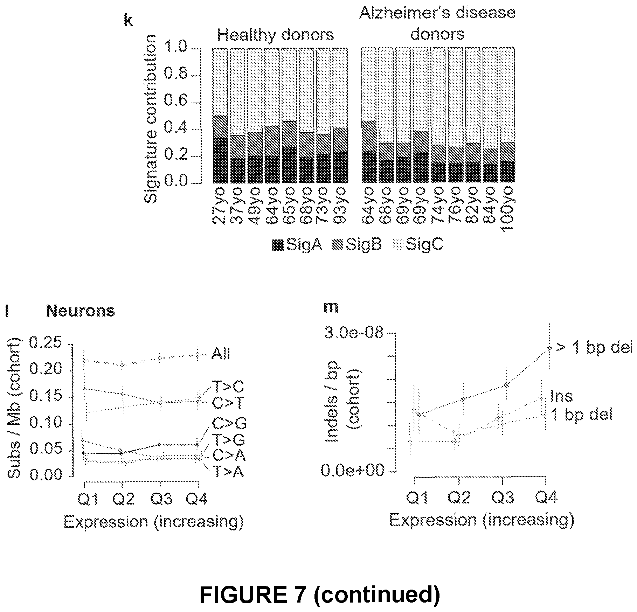

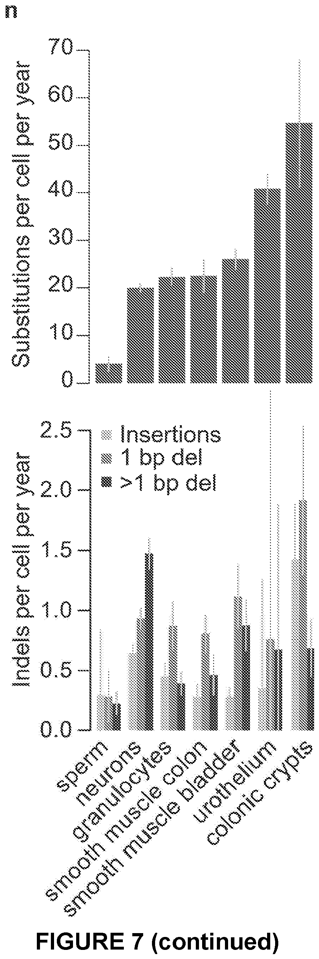

**Figure 7:** Mutation landscape in smooth muscle and neurons. **A)** and **B)** Substitutions and indels per cell in smooth muscle from 10 donors spanning different ages; point estimate confidence intervals and linear mixed model confidence intervals as in **Figure 2A.** Intercept and slope for substitutions: 239.3 [-211.5-653.9] and 20.7 [13.6-28.0], respectively. Intercept and slope for indels: 50.0 [2.6-97.2] and 1.3 [0.4-2.3, respectively. **C)** and **D)** Substitution and indel spectra in smooth muscle. **E)** Signature decomposition using granulocytes, colonic crypts, smooth muscle and neurons substitution data. **F)** Exposure to signatures A, B and C in smooth muscle for each donor and organ of origin. **G)** and **H)** Substitution and indel accumulation per neuron throughout life; point estimate confidence intervals as in **Figure 2A;** grey area shows simple linear model 95% confidence intervals. Intercept and slope for substitutions: 210.5 [-26.9-448.0] and 17.1 [13.7-20.5], respectively. Intercept and slope for indels: 45.9 [-10.2-102.0] and 2.5 [1.7-3.3], respectively. **I)** and **J)** Substitution and indel spectra in neurons from healthy and Alzheimer's Disease donors. **K)** Contribution of signatures A, B and C in neurons. **L)** and **M)** Substitution and indel rates in the whole cohort for genes in four quantiles of expression, showing different types of substitutions and indels. Lines show Poisson 95% confidence intervals. **N)** Overall comparison of substitution and indel rates in different cell types.

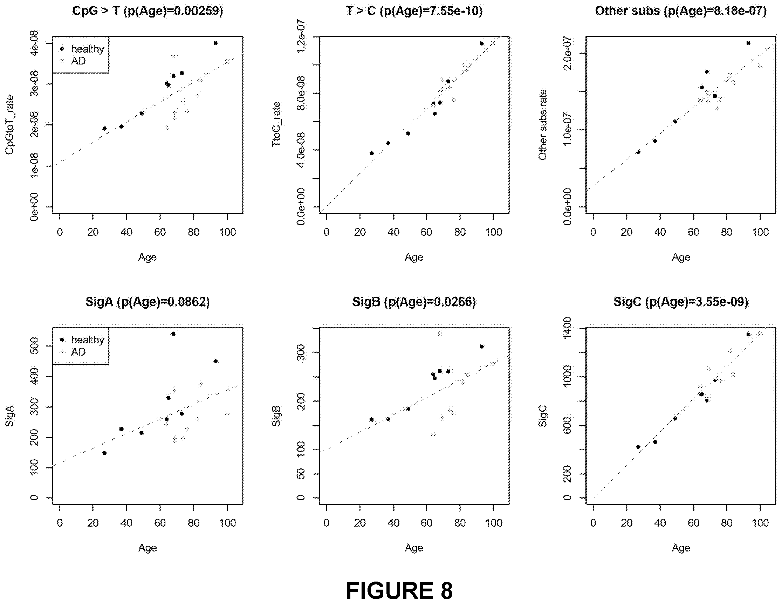

**Figure 8:** Top panels show the accumulation of C>T at CpG, T>C and other substitutions in the neuron cohort throughout lifespan. *P* values on top show the significance of the slope, i.e. whether there is an increase of mutations with age. Bottom panels show the accumulation of mutations attributed to signatures A, B, and C throughout lifespan.

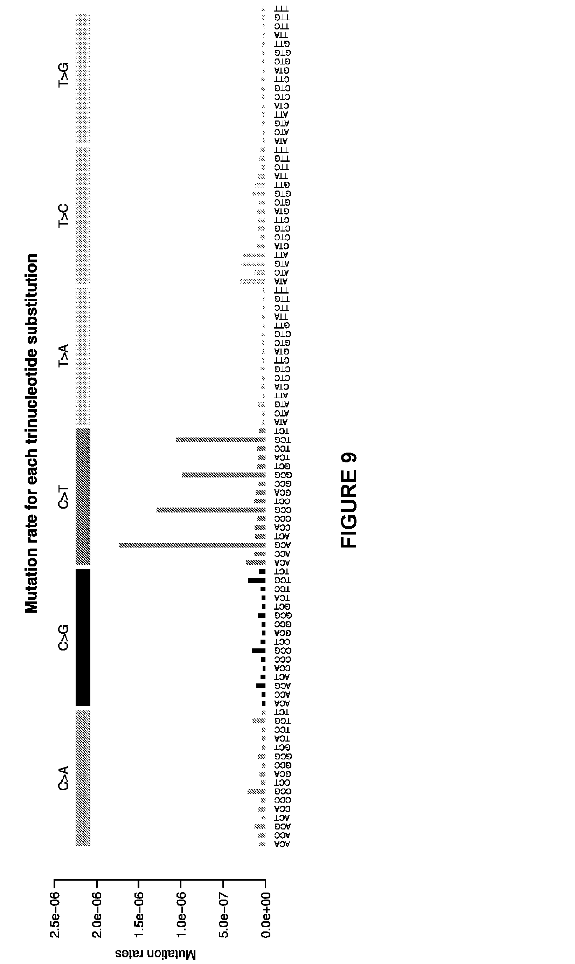

**Figure 9:** Non-normalised mutation rate for each of the 96 trinucleotide substitution types in neurons.

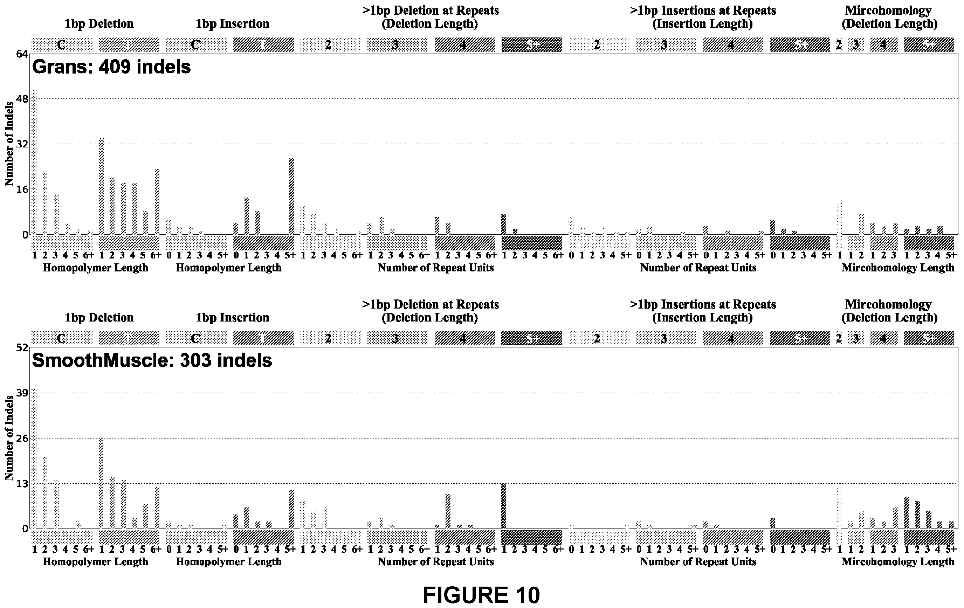

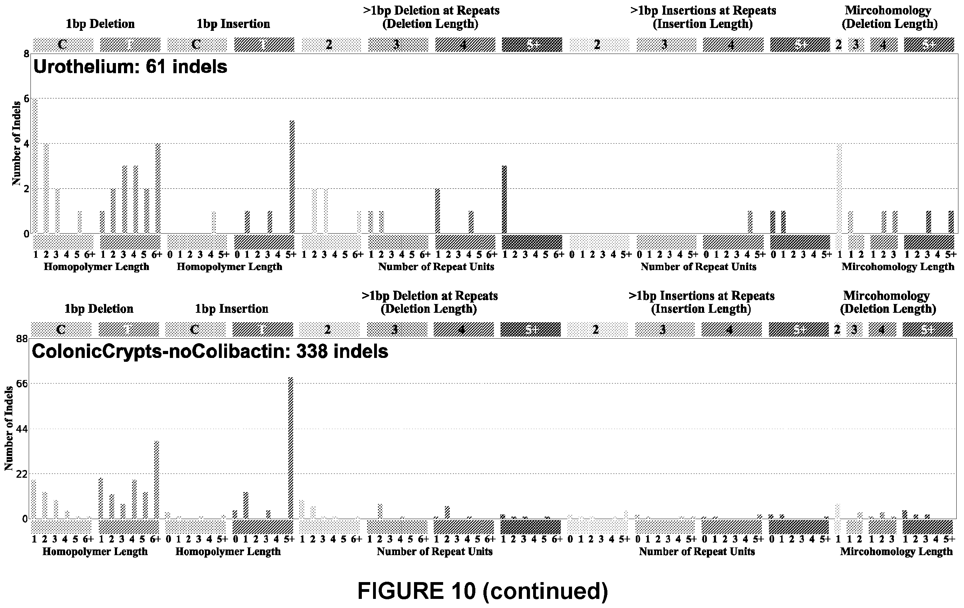

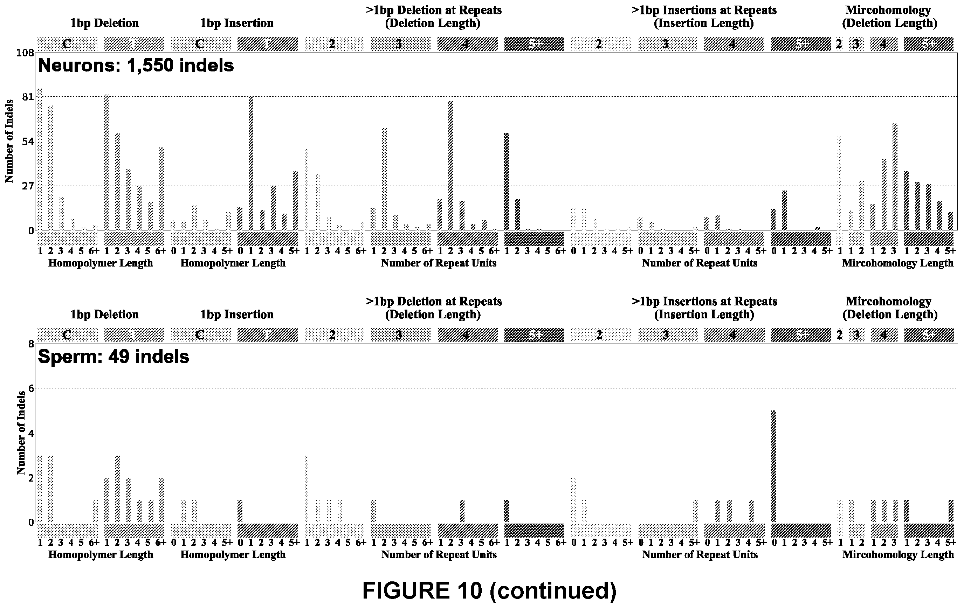

**Figure 10:** Indel profiles obtained from NanoSeq data for granulocytes, colonic crypts (excluding samples with colibactin signature), smooth muscle, neurons, urothelium and sperm.

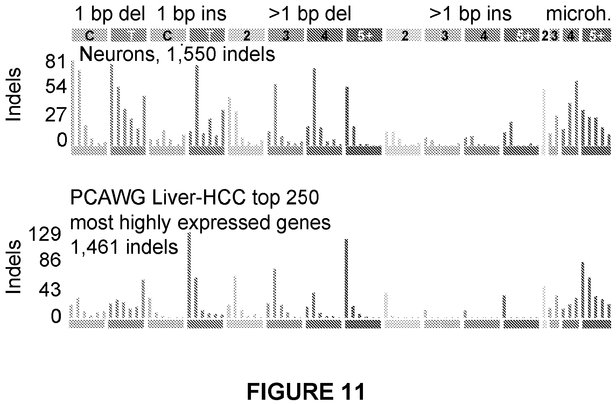

**Figure 11:** Top panel: indel profile for neurons. Bottom Panel: indel profile for the 250 most highly expressed genes in liver hepatocellular carcinoma.

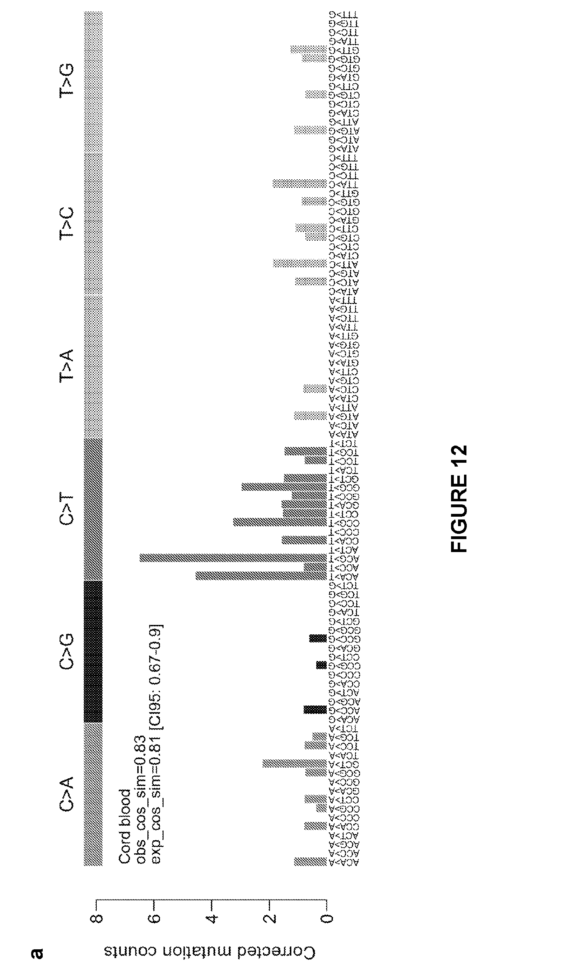

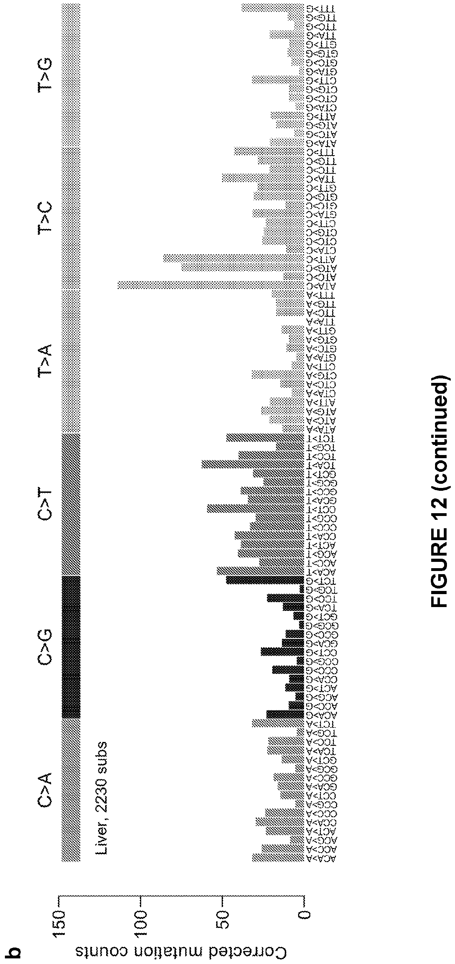

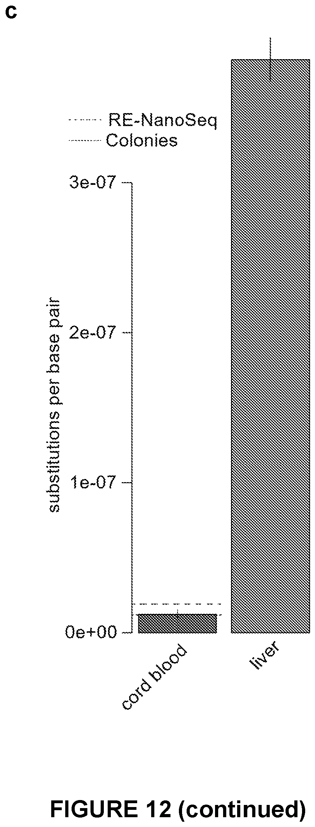

**Figure 12:** Somatic mutation spectra and burden using whole-exome NanoSeq on cord blood and chronic liver disease samples. **A)** Mutation spectrum of a cord blood sample projected into the whole-genome trinucleotide composition. The high cosine similarity with the mutation spectrum of single-cell derived colonies is consistent with a very low error rate (Abascal *et al.* (2021)). **B)** Aggregated mutation spectrum of whole-exome NanoSeq on several polyclonal liver biopsies from a patient with non-alcoholic fatty liver disease. This experiment detected 3 likely pathogenic variants across these samples (**Table 2**), including two in *FOXO1* and one in *CIDEB* (Ng *et al.* (2021) doi: https://doi.org/10.1038/s41586-021-03974-6). **C)** Estimated mutation burden for the cord blood and liver samples. The low mutation burden of the cord blood sample is in line with the known mutation rate of cord blood cells from single-cell derived colonies, confirming that the error rate of whole-exome NanoSeq in this experiment was $<5\times10^{-9}$.

## DETAILED DESCRIPTION OF THE INVENTION

The invention relates to a method of generating a nucleic acid library for sequencing, such as DNA sequencing, with error rates <1 error per 100 million base pairs. According to a first aspect of the invention, there is provided a method of generating a nucleic acid library for sequencing, said method comprising the steps of:

(i) fragmenting a nucleic acid composition to generate fragmented nucleic acid molecules with blunt ends;
(ii) introducing dideoxy nucleotides at internal nick sites present in the fragmented nucleic acid molecules and tailing to generate tailed nucleic acid fragments; and
(iii) adding sequencing adaptors to the tailed nucleic acid fragments to generate a nucleic acid library.

In one embodiment, fragmenting the nucleic acid composition in step (i) comprises using one or more blunt/non-cohesive end-generating endonuclease. Thus, in one aspect of the invention the method comprises the steps of:

(i) fragmenting a nucleic acid composition using a blunt/non-cohesive end-generating endonuclease to generate fragmented nucleic acid molecules with blunt ends;
(ii) introducing dideoxy nucleotides at internal nick sites present in the fragmented nucleic acid molecules and tailing the fragmented nucleic acid molecules to generate tailed nucleic acid fragments; and
(iii) adding sequencing adaptors to the tailed nucleic acid fragments to generate a nucleic acid library.

Thus, disclosed herein is nanorate sequencing (NanoSeq), a method of generating a nucleic acid library for sequencing which provides reliable detection of mutations in single DNA molecules. Other methods of duplex sequencing are known in the art and include BotSeqS, a whole-genome duplex sequencing protocol developed for the study of mutagenesis in human tissues (Hoang *et al.* (2016) doi: https://doi.org/10.1073/pnas.1607794113). Such methods rely on duplex consensus sequencing principles, where both strands of DNA are differently tagged, amplified and sequenced. By sequencing multiple copies from each strand of a single DNA molecule, these protocols can remove both sequencing errors (present in individual reads) and PCR errors introduced during the library preparation stage (restricted to copies of one of the two strands) - see **Figure 1A.** Under the assumption that errors in reads from both strands are independent, duplex sequencing could achieve a theoretical error rate of less than $1\times10^{-9}$ errors/bp, the probability of two early and complementary PCR errors in both strands. Given that this theoretical error rate limit is lower than the typical somatic mutation loads of human tissues, these technologies raise the possibility of quantifying mutation rates in any cell type of interest, independently of its clonal architecture. However, duplex sequencing methods developed to date, including BotSeqS, suffer from the same limitations as mentioned above, namely mapping errors and the accidental copying of errors between strands, due to the techniques employed in library preparation. These limitations therefore lead to loss of independence between strands and could limit the accuracy of duplex sequencing.

Furthermore, the inventors have evaluated the performance of the existing BotSeqS protocol (**Figures 1A-1B**) by first analysing a sample of circulating granulocytes from a 59-year-old donor from whom 110 single cell-derived blood colonies had been subjected to whole-genome sequencing (Lee-Six *et al.* (2018) doi: https://doi.org/10.1038/s41586-018-0497-0). It was found that the estimates of mutation burden per cell from BotSeqS were two-fold higher than those from single cell-derived colonies (**Figure 1C**) and the substitution profiles were dissimilar (cosine similarity of 0.71; **Figure 1D**), with increased C>A and C>G substitution rates. To determine whether the excess substitutions were artefacts introduced during library preparation, the distribution of substitutions across the reads was analysed. This revealed a large excess of G>T/C substitutions near the 5' ends of DNA fragments, and an imbalance over C>A/G substitutions that affected the entire read length (**Figure 1E**). These substitution imbalances are incompatible with real mutations and reflect errors introduced during library preparation (Costello *et al.* (2013) doi: https://doi.org/10.1093/nar/gks1443) and Chen *et al.* (2017) doi: https://doi.org/10.1126/ science.aai8690). The same imbalances, together with an additional C>T imbalance, were then confirmed to be present in the original BotSeqS publication (Hoang *et al.* (2016); **Figure 1E**). Imbalances were stronger at read ends but extended deep into the reads.

Without wishing to be bound by any particular theory, it is reasoned that these artefacts seen in BotSeqS could be caused by end repair during library preparation. Nucleic acid (e.g. DNA) fragmentation by sonication requires end repair to generate blunt-ended fragments, which is typically achieved by exonuclease digestion of 3' ends and by copying 5' overhangs. Thus, damaged bases on 5' overhangs can lead to damage in one strand being fixed as an apparent mutation in both strands (**Figure 1B**), violating the error correction mechanism of duplex sequencing. Evidence was also found that internal nicks can prime extension by DNA polymerases during end repair. Therefore, in one embodiment, the method comprises the step of fragmenting a nucleic acid composition using a blunt/non-cohesive end-generating endonuclease to generate fragmented nucleic acid molecules. For ease of identification, the step of generating fragmented nucleic acid molecules is referred to herein as step (i). The terms "blunt end" and "non-cohesive end" as used interchangeably herein refer to ends of nucleic acid molecules, such as DNA strands, at which both strands terminate in a base pair. This is in contrast to an overhanging, cohesive or sticky-end which comprises a stretch of unpaired nucleotides.

It will therefore be appreciated that, in certain embodiments, using a blunt/non-cohesive end-generating endonuclease in step (i) prevents the need for end-repair of the fragmented nucleic acid molecules. Using a blunt/non-cohesive end-generating endonuclease in step (i) also prevents the need for the removal of overhangs from or trimming of the fragmented nucleic acid molecules using an exonuclease enzyme. Such repair or removal of overhangs is necessary when sonication or sticky end-generating enzymes are used. As mentioned hereinbefore, by avoiding the need for end-repair and the copying of 5' overhangs to generate blunt-ended nucleic acid fragments, the independence of each nucleic acid strand is maintained and the introduction of double-stranded errors is avoided. Thus, in certain embodiments, generating fragmented nucleic acid molecules in step (i) produces nucleic acid fragments with blunt/non-cohesive ends. In further embodiments, the nucleic acid fragments generated in step (i) do not comprise overhanging, cohesive or sticky ends. Thus, in some embodiments the nucleic acid fragments generated in step (i) comprise blunt ends. The need for end-repair using a DNA polymerase can also be avoided and the independence of each nucleic acid strand will be maintained when overhangs, such as overhangs in nucleic acid fragments generated by sonication, are removed/trimmed using an exonuclease enzyme.

In one embodiment, the blunt/non-cohesive end-generating endonuclease is a blunt/non-cohesive end-generating restriction endonuclease having a 4 base-pair recognition site. In a further embodiment, the blunt/non-cohesive end-generating endonuclease is a blunt/non-cohesive end-generating restriction endonuclease which is not impaired by overlapping CpG methylation. Such restriction endonucleases suitable for use in the methods described herein are well known in the art and would be easily recognised by the skilled person. Examples include AluI, HaeIII, HindII, SmaI and HpyCH4 (including subtypes thereof, such as HpyCH4III, HpyCH4IV or HpyCH4V). It will also be appreciated that, for the purpose of preparing a nucleic acid library for sequencing, restriction endonuclease enzymes yielding sufficient coverage of the whole genome to be sequenced are preferred. For example, the coverage of AluI has been determined by the inventors using *in silico* digestion to yield 27% of the nuclear genome in fragments between 250-500 base pairs (and 150 base pairs sequenced read pairs), 28.3% of coding sequences and 26.1% of the mitochondrial genome. The coverage of HpyCH4V has been determined to be 28.7% of the nuclear genome, 28% of coding sequences and 10.9% of the mitochondrial genome (see **Table 1**). Therefore, in one embodiment, the restriction endonuclease provides enough coverage of the nuclear genome and coding sequences. In another embodiment, the restriction endonuclease has coverage of the nuclear genome, coding sequences and the mitochondrial genome. In a further embodiment, multiple different restriction endonuclease enzymes may be used to prepare nucleic acid libraries for sequencing. Such use of multiple different restriction endonuclease enzymes may be in separate libraries which are then pooled or may be in the same library in combination. Thus, in a yet further embodiment, a first restriction endonuclease having partial coverage of the nuclear genome and coding sequences is used to prepare a first nucleic acid library and a second restriction endonuclease having different partial coverage of the nuclear genome and coding sequences is used to prepare a second nucleic acid library. Said first and second nucleic acid libraries may then be pooled for sequencing. In another embodiment, a first restriction endonuclease having partial coverage of the nuclear genome and coding sequences and a second restriction endonuclease having different partial coverage of the nuclear genome and coding sequences are used in combination to prepare a nucleic acid library for sequencing. According to this embodiment, pooling of differentially prepared nucleic acid libraries is not required for sequencing.

Table 1 - *In silico* digestion of restriction endonucleases, showing fraction of genome covered assuming full digestion, size selection between 250 and 500bps and read lengths of 150bps.

| Restriction enzyme | Restriction site | Nuclear genome | % cov* | Coding sequences | % cov* | Mitochondrial genome | % cov* |
|---|---|---|---|---|---|---|---|
| AluI | AGCT | 810,043,421 | 27.0 | 9,990,036 | 28.3 | 4,171 | 26.1 |
| BfaI | CTAG | 546,344,998 | 18.2 | 5,205,874 | 14.7 | 5,527 | 34.5 |
| CviAII | CATG | 810,011,070 | 27.0 | 9,490,545 | 26.9 | 2,061 | 12.9 |
| CviJI | RGCY, RGCR, YGCR | 60,139,430 | 2.0 | 256,061 | 0.7 | NA | NA |
| CviKI-1 | RGCY | 433,565,956 | 14.5 | 2,780,668 | 7.9 | 586 | 3.7 |
| CviQI | GTAC | 321,512,873 | 10.7 | 4,307,494 | 12.2 | 840 | 5.3 |
| DpnII | GATC | 510,329,959 | 17.0 | 6,202,629 | 17.6 | 877 | 5.5 |
| FatI | CATG | 810,011,070 | 27.0 | 9,490,545 | 26.9 | 2,061 | 12.9 |
| HaeIII | GGCC | 445,692,040 | 14.9 | 5,673,040 | 16.1 | 2,655 | 16.6 |
| HpyCH4V | TGCA | 862,397,747 | 28.7 | 9,886,638 | 28.0 | 1,739 | 10.9 |
| MluCI | AATT | 690,934,232 | 23.0 | 6,355,541 | 18.0 | 3,493 | 21.8 |
| MseI | TTAA | 705,362,365 | 23.5 | 6,117,438 | 17.3 | 4,474 | 28.0 |
| MspI | CCGG | 83,530,412 | 2.8 | 3,314,212 | 9.4 | 1,800 | 11.3 |
| TaqI | TCGA | 53,681,028 | 1.8 | 1,474,987 | 4.2 | 1,428 | 8.9 |

* "% cov" refers to the percent coverage of the indicated portion of the genome (either nuclear (also referred to as "whole" herein), coding sequences or the mitochondrial genome).

Thus, in one embodiment, the blunt/non-cohesive end-generating restriction endonuclease is selected from one or both of: AluI and HpyCH4, such as HpyCH4III, HpyCH4IV or HpyCH4V. As will be appreciated from the disclosures hereinbefore, such use of one or both restriction endonucleases provides partial coverage across the nuclear genome, coding sequences and the mitochondrial genome. In one embodiment, the restriction endonuclease is AluI and provides partial coverage of the nuclear genome, coding sequences and the mitochondrial genome. In another embodiment, the restriction endonuclease is HpyCH4, such as HpyCH4III, HpyCH4IV or HpyCH4V, and provides partial coverage of the nuclear genome and coding sequences. In a further embodiment, the one or more restriction endonucleases are used for the preparation of separate nucleic acid libraries which are then pooled prior to sequencing. In an alternative embodiment, the one or more restriction endonucleases are used in combination for the preparation of the same nucleic acid library. Thus, in a yet further embodiment, the blunt/non-cohesive end-generating restriction endonuclease is selected from both of: AluI and HpyCH4, such as HpyCH4III, HpyCH4IV or HpyCH4V.

In alternative embodiments, fragmenting the nucleic acid composition in step (i) comprises removing overhangs from fragmented nucleic acid molecules using one or more exonuclease enzyme to generate fragmented nucleic acid molecules with blunt ends. Thus, step (i) may be separated into step (i a) which comprises initial fragmentation of the nucleic acid composition and step (i b) which comprises removal of overhangs to generate fragmented nucleic acid molecules with blunt ends. In some embodiments fragmenting the nucleic acid composition in step (i)/(i a) comprises mechanical fragmentation. In one embodiment, fragmenting the nucleic acid composition in step (i)/(i a) comprises sonication. Therefore, according to a further aspect of the invention the method comprises the steps of:

(i a) fragmenting a nucleic acid composition by sonication to generate fragmented nucleic acid molecules;
(i b) removing overhangs from the fragmented nucleic acid molecules using one or more exonuclease enzyme to generate fragmented nucleic acid molecules with blunt ends;
(ii) introducing dideoxy nucleotides at internal nick sites present in the fragmented nucleic acid molecules and tailing to generate tailed nucleic acid fragments; and

(iii) adding sequencing adaptors to the tailed nucleic acid fragments to generate a nucleic acid library.

In other embodiments, fragmenting the nucleic acid composition in step (i)/(i a) comprises mechanical shearing. It will be readily appreciated that in contrast to when a restriction endonuclease which requires the presence of a restriction site sequence is used, sonication and/or shearing of the nucleic acid composition which are sequence-independent, generate nucleic acid fragments representing complete coverage across the genome, including complete coverage of the nuclear genome, coding sequences and the mitochondrial genome. As described hereinbefore, trimming or removal of overhangs using exonuclease enzymes maintains the independence of each nucleic acid strand and any end-repair by copying 5' overhangs, such as using a DNA polymerase, is avoided. Thus, in one embodiment using an exonuclease enzyme to generate fragmented nucleic acid molecules with blunt ends prevents the need for end-repair of the fragmented nucleic acid molecules, avoiding the introduction of double-stranded errors.

In one embodiment, the one or more exonuclease enzyme is Mung Bean nuclease. Mung Bean nuclease is derived from the sprouts of the mung bean and removes nucleotides from single-stranded DNA and RNA in a step wise manner. It is therefore a specific nucleic acid exonuclease that degrades single-stranded extensions/overhangs from the ends of DNA and RNA while leaving double-stranded nucleic acid molecules intact. Following degradation of single-stranded extensions or overhangs, Mung Bean nuclease leaves blunt ends to which adaptors, such as sequencing adaptors, can be added by ligation.

It has also been found by the inventors that, when using restriction endonuclease enzymes to fragment a nucleic acid composition according to step (i), A-tailing of nucleic acid fragments prior to ligation of sequencing adaptors in standard duplex sequencing protocols led to increased levels of C>A and T>A at restriction sites and the profile of single-strand consensus, typically caused by DNA damage, showed an increased amount of G>A, C>A and T>A. It is hypothesised that this is due to a multi-step mechanism involving: nicking of the nucleic acid duplex by restriction enzymes; 3' to 5' exonuclease activity or pyrophosphorolysis activity of the dNTP located 3' of the nick; incorporation of dATP opposite C, G or A during A-tailing (causing G>A, C>A or T>A, respectively); and subsequent sealing of the internal nick during adaptor ligation. Nicking may also occur during fragmentation of the nucleic acid molecules by sonication and/or shearing. Therefore, to block nucleic acid molecules with internal nicks or gaps, dATP may be replaced with a mixture of dATP and ddBTP dideoxy nucleotides (ddCTP, ddGTP, ddTTP) during A-tailing. Without wishing to be bound by any particular theory, when a mixture of dATP and ddBTP dideoxy nucleotides is used for A-tailing, DNA polymerase extension will proceed until a ddBTP which has been incorporated at an internal nick site is reached. The ddBTP then blocks said extension at the internal nick, leaving the nucleic acid fragment unusable by preventing PCR amplification of the affected strand. The results presented herein show that the incorporation of ddBTPs successfully removed artefacts caused by A-tailing (see **Figure 2**).

Therefore, in one embodiment, the method comprises the step of introducing dideoxy nucleotides at internal nick sites present in the fragmented nucleic acid molecules with blunt ends and tailing the fragmented nucleic acid molecules to generate tailed nucleic acid fragments. For ease of identification such a step of introducing dideoxy nucleotides at internal nick sites is referred to herein as step (ii). The terms "nick" and "gap" as used interchangeably herein refer to the absence of a nucleotide at a position within one strand of the nucleic acid. Such nick/gap will be positioned opposite a nucleotide on the other strand of the nucleic acid which is complementary to the nucleotide previously occupying the position represented by the nick/gap. As will be appreciated by the use of the term "internal", such nicks/gaps are not found at the ends of the nucleic acid fragment and are found at least one nucleotide position in from either the 5' or 3' end of a nucleic acid strand.

As such, in one embodiment, introducing dideoxy nucleotides at internal nick sites in step (ii) prevents extension of the internal nick and results in an unamplifiable nucleic acid strand. As mentioned hereinbefore, preventing the extension of the internal nick blocks the extension by DNA polymerase and results in an unamplifiable nucleic acid stand. The failure to amplify one of the strands prevents obtaining duplex coverage (i.e. sequencing reads from both strands) for said nucleic acid fragment having a nick into which a dideoxy nucleotide has been incorporated.

It will be readily appreciated that the specific identity of dideoxy nucleotides to be used for incorporation at internal nick sites of the nucleic acid fragments during tailing will depend on the identity of the nucleotides used for said tailing. For example, if the nucleic acid fragment is A-tailed, the dideoxy nucleotides will be dideoxy non-A nucleotides, such as ddBTPs. Alternatively, if the nucleic acid fragment is C-tailed, the dideoxy nucleotides will be dideoxy non-C nucleotides, such as ddDTP, if the nucleic acid fragment is G-tailed, the dideoxy nucleotides will be dideoxy non-G nucleotides, such as ddHTP, or if the nucleic acid fragment is T-tailed, the dideoxy nucleotides will be non-T nucleotides, such as ddVTP. Therefore, in one embodiment, the dideoxy nucleotides introduced in step (ii) are dideoxy non-A nucleotides, such as ddBTPs. According to this embodiment, the tailing of the fragmented nucleic acid molecules in step (ii) comprises A-tailing. In a further embodiment, the dideoxy nucleotides introduced in step (ii) are dideoxy non-C nucleotides, such as ddDTP. According to this further embodiment, the tailing of the fragmented nucleic acid molecules in step (ii) comprises C-tailing. In another embodiment, the dideoxy nucleotides introduced in step (ii) are dideoxy non-G nucleotides, such as ddHTP. According to this embodiment, the tailing of the fragmented nucleic acid molecules in step (ii) comprises G-tailing. In a still further embodiment, the dideoxy nucleotides introduced in step (ii) are dideoxy non-T nucleotides, such as ddVTP. According to this further embodiment, the tailing of the fragmented nucleic acid molecules in step (ii) comprises T-tailing. Thus, in one embodiment, the tailing of the fragmented nucleic acid molecules in step (ii) comprises A-tailing and the dideoxy nucleotides introduced are dideoxy non-A nucleotides, such as ddBTPs. In another embodiment, the tailing of the fragmented nucleic acid molecules in step (ii) comprises C-tailing and the dideoxy nucleotides introduced are dideoxy non-C nucleotides, such as ddDTP. In a further embodiment, the tailing of the fragmented nucleic acid molecules in step (ii) comprises G-tailing and the dideoxy nucleotides introduced are dideoxy non-G nucleotides, such as ddHTP. In a yet further embodiment, the tailing of the fragmented nucleic acid molecules in step (ii) comprises T-tailing and the dideoxy nucleotides introduced are dideoxy non-T nucleotides, such as ddVTP.

In order to generate a nucleic acid library for sequencing, sequencing adaptors are added to the tailed nucleic acid fragments. Therefore, in one embodiment, the method comprises the step of adding sequencing adaptors to the tailed nucleic acid fragments to generate a nucleic acid library. For ease of identification, such as step of adding sequencing adaptors is referred to herein as step (iii). Such sequencing adaptors allow automated high throughput sequencing, such as next-generation sequencing (NGS). For example, such high throughput sequencing devices that are compatible with these adaptors include, but are not limited to Solexa (Illumina), the 454 System, and/or the ABI SOLiD. For example, the method may include using universal primers in conjunction with poly-A tails. In one embodiment, the adaptors comprise Illumina sequencing adaptor sequences.

The sequencing adaptors may comprise a barcode sequence (known as duplex sequencing adapters). Thus, in one embodiment, the sequencing adaptors are duplex sequencing adaptors comprising a barcode sequence. Barcode sequences are necessary to identify copies from a single molecule when the read start and ends do not offer sufficient diversity as unique molecular identifiers (e.g. when using restriction enzyme digestion or deep sequencing). Therefore, in one embodiment, the duplex sequencing adaptors are barcoded. The presence of barcode sequences, such as 3 nucleotide barcode sequences, allows for the unique identification of sequencing reads originating from either of the strands of the nucleic acid composition and/or the identification of duplicate reads originating from PCR duplication during library preparation. Thus, in a further embodiment, the barcode is unique to one strand of the nucleic acid composition. In a yet further embodiment, the barcoded duplex sequencing adaptors comprise nucleotide barcode sequences made up of several random nucleotides, such as a 3 to 8 nucleotide tag, e.g. a 3 nucleotide tag. These barcodes uniquely tag the end of nucleic acid fragments which would otherwise be indistinguishable when using blunt/non-cohesive end-generating restriction endonuclease enzymes which generate independent nucleic acid fragments having the same coordinates used for mapping during subsequent analysis of sequencing data or when sequencing a library to high depth. In contrast, the use of mechanical fragmentation and removal of overhangs using one or more exonuclease enzyme generates a wider diversity of breakpoints that can be used to distinguish copies from an original molecule of DNA. Such breakpoints can be used as unique molecular identifiers alone or in combination with a barcode in the duplex sequencing adapters. In a further embodiment, following addition of duplex sequencing adaptors, the nucleic acid molecules within the library comprise the sequence NNNTCA or NNNXTCA when the restriction endonuclease HpyCH4V has been used to fragment the nucleic acid composition (HpyCH4V cuts at the sequence TGCA). According to this embodiment, NNN represents a random 3 nucleotide barcode (e.g. a 3 nucleotide tag), T is the adaptor overhang and X is a 'spacer' nucleotide designed to increase nucleotide diversity during sequencing.

The nucleic acid library may be subject to selective enrichment in order to enrich one or more genomic region of interest prior to sequencing. Thus, in some embodiments the method additionally comprises the step of: (iv) selectively enriching nucleic acids corresponding to one or more genomic region of interest to generate an enriched nucleic acid library. Examples of genomic regions of interest include, but are not limited to, specific genes or a panel of specific genes (e.g. a gene of particular interest or a panel of genes of particular interest), coding sequences (CDSs; e.g. protein coding sequences), exonic sequences (including the whole exome) and intronic sequences. In one embodiment, the one or more genomic region of interest is one or more coding sequences (CDSs) of coding genes, such as protein coding sequences. In a further embodiment, the one or more genomic regions of interest are exonic sequences, such as the exome. Thus, in a particular embodiment the genomic region of interest is the entire/whole exome, which comprises all protein coding sequences (e.g. for the purpose of unbiased discovery of disease-associated coding mutations). In a yet further embodiment, the one or more genomic region of interest is one or more specific gene of interest. Such one or more gene of interest may be a single gene or a group/panel of genes (e.g. a group of genes known to be associated with growth and/or cancer, such as a group/panel of oncogenes or tumour suppressor genes). Thus, in one embodiment the one or more genomic region of interest is a panel of genes.

In order to selectively enrich one or more genomic region of interest, any suitable method known in the art may be used. For example, oligonucleotide probes having sequences which are complementary to the genomic region(s) of interest may be used. Such oligonucleotide probes therefore bind to sequences comprising the genomic region(s) of interest, allowing them to be enriched or isolated from sequences in the nucleic acid library which do not comprise the genomic region(s) of interest. In one embodiment, the method is for generating a nucleic acid library for exome (e.g.

whole-exome) sequencing and selectively enriching nucleic acids corresponding to genomic regions of interest in step (iv) comprises using oligonucleotide probes which bind to the coding sequences (CDSs). Non-limiting examples of sets of oligonucleotide probes which bind to CDSs include the xGen Exome Research Panel from Integrated DNA Technologies (IDT), SureSelect probes from Agilent and the gene or exome enrichment panels from Twist Bioscience.

Sequencing

In a particular embodiment, the nucleic acid library is for duplex sequencing. As described hereinbefore, duplex sequencing utilises differential tagging, amplification and sequencing of each strand of the nucleic acid molecule to generate sequencing data for each strand and a duplex consensus sequence. This duplex consensus, created by sequencing multiple copies from each strand of a single nucleic acid molecule, removes both sequencing errors (present in individual reads) and PCR errors introduced during the library preparation stage (restricted to copies of one of the two strands). Under the assumption that errors in reads from both strands are independent, such duplex sequencing can achieve a theoretical error rate of less than $1 \times 10^{-9}$ errors/bp, the probability of two early and complementary PCR errors in both strands. As this theoretical error rate limit is lower than the typical somatic mutation rates of human tissues, it is possible to quantify mutation rates in any cell type of interest, independently of its clonal architecture. However, in previous implementations of duplex sequencing, errors from one strand can be copied into the complementary strand during library preparation, violating the independence of both strands and limiting the accuracy of the method. The present method, unlike previous duplex sequencing protocols, maintains the independence between strands, and thus achieves a higher sequencing accuracy.

It will be appreciated that references herein to "sequencing" are not limited to any particular technique for sequencing the nucleic acid library. For example, sequencing may be performed using a platform which comprises use of a polymerase chain reaction (PCR), such as a next generation sequencing (NGS) platform. Such platforms include sequencing-by-synthesis platforms such as Illumina's TruSeq technology which supports massively parallel sequencing using a proprietary reversible terminator-based method that enables detection of single bases as they are incorporated into growing DNA strands. Further examples of suitable NGS platforms include, but are not limited to: the Roche 454 platform (i.e. Roche 454 GS FLX) which employs pyrosequencing, whereby a chemiluminescent signal indicates base incorporation and the intensity of signal correlates to the number of bases incorporated through homopolymer reads; Applied Biosystems' SOLiD system (i.e. SOLiDv4); Illumina's GAIIx, HiSeq 2000, NovaSeq, HiSeq4000 and MiSeq sequencers; Life Technologies' Ion Torrent semiconductor-based sequencing instruments which use a strategy similar to sequencing-by-synthesis but detect signal by the release of hydrogen ions resulting from the activity of DNA polymerase during nucleotide incorporation; Pacific Biosciences' PacBio RS; Sanger's 3730xl; and nanopore-based sequencing platforms such as those in which the nanopore is constructed from a metal, polymer or plastic material or Oxford Nanopore Technologies' organic-type nanopore-based system which mimics the situation of the cell membrane and protein channels in living cells.

The efficiency and cost-effectiveness of duplex sequencing depends on optimising the duplicate rate to maximise the number of read bundles (defined as a family of PCR duplicates) with at least 2 duplicate reads from each original strand. Too high duplicate rates result in few read bundles of unnecessarily large sizes, whereas too low duplicate rates result in many read bundles with few having two or more read pairs from each strand.

To maximise the efficiency of the protocol, the relationship between the number of DNA molecules in the library (library complexity) and the resulting duplicate rate as a function of the number of read pairs sequenced was studied analytically and empirically. It was found that optimal duplicate rates and optimal efficiency can be ensured across a wide range of samples by quantifying the concentration of the DNA library using a fraction of the library and using this information to amplify an optimal amount of the remaining library for DNA sequencing. If negligible PCR biases are assumed, with copies from all original ligated DNA fragments represented in equimolar amounts in the amplified library, the bundle size distribution of observed reads can be modelled as a zero-truncated Poisson distribution. Let r (sequence ratio) be the ratio between the number of sequenced reads and the number of amplifiable DNA fragments in the original library. The mean read bundle size (m) can then be estimated as the mean of the zero-truncated Poisson distribution:

$$m = \frac{r}{1-e^{-r}}$$ . This parameter then enables a simple estimation of the duplicate rate of a library (d, defined as the fraction of reads that are duplicate copies, and identified as reads having the same barcodes and the same 5' and 3' coordinates): $$d = \frac{m-1}{m} = 1 - \frac{1}{m} = 1 - \frac{1-e^{-r}}{r}$$ .

The efficiency of a duplex sequencing library (E) can be defined as the ratio between the number of base pairs with duplex coverage (bundles with $\geq 2$ reads from both strands) and the number of base pairs sequenced. This can be

modelled as: $E = \dfrac{P(x \geq 2; \frac{r}{2})^2}{m}$ , where the numerator is the probability of a read bundle having at least two reads from both strands (i.e. usable bundles), based on the zero-truncated Poisson distribution (denoted as $P$), and the denominator is the sequence investment in each read bundle (i.e. the average read bundle size). Based on this equation, it can be estimated numerically that the optimal duplicate rate is ~81% and that duplicate rates between 65-90% would yield $\geq$80% of the maximum attainable efficiency. In terms of r, the optimum $r$ is 5.1 read pairs sequenced per original DNA fragment ($r_{opt}$), with values within 2.7-9.6 yielding $\geq$80% of the maximum efficiency. Knowing the concentration of a NanoSeq (or BotSeqS) library in fmol/$\mu$l (estimated using a qPCR reaction on an aliquot of the library), $r_{opt}$ can be used to calculate the volume of library that needs to be amplified to yield optimal duplicate rates (i.e. maximum duplex efficiency), as a

$$fmol_{opt} = \dfrac{N}{f \, r_{opt}}$$

function of the desired amount of raw sequencing: . Here, $N$ is the number of paired-end reads that will be sequenced and $f$ is the number of DNA fragments per fmol of library (referring specifically to ligated and amplifiable fragments within the size selection range). Using an initial set of libraries, a range of library inputs (fmol) was compared to the estimated number of unique molecules in the library inferred from the sequencing data (using Piccard's software). This analysis revealed that, for a particular choice of restriction enzyme and size selection conditions, $f$ approximately equated to $10^8$ fragments/fmol.

Using the above equation, the efficiency of NanoSeq can be optimised independently of the input amount of DNA in a given sample. For example, ~0.3 fmols of library yielded optimal duplicate rates when using 150 million 150bp paired-end reads, which are the equivalent of ~15x coverage in standard human whole-genome sequencing. -0.6 fmol yielded optimal efficiency when using 300 million reads (30x whole-genome equivalent). Note that, as predicted by the equations above, deviations ~2-fold from $r_{opt}$ still yield high efficiency. Using these equations, near-optimal duplicate rates from a wide diversity of samples were reliably obtained. Overall, it was found that ~30x of standard sequencing output (~300$\times$10$^6$ 150bp paired-end reads) yielded approximately 3Gb of high-accuracy duplex coverage (a haploid genome equivalent) after application of all computational filters.

Therefore, in one embodiment, the nucleic acid library is diluted to a concentration which maximises duplex coverage as predicted using an analytical and empirical model. Thus, in some embodiments, the nucleic acid library is diluted to a concentration which is determined by and is dependent on the number of paired-end reads which are equivalent to a desired equivalent duplex coverage of the whole-genome. In further embodiments, the analytical and empirical model is as described hereinbefore. In a yet further embodiment, the duplex coverage is between around 15x and 30x whole-genome equivalent and the nucleic acid library is diluted to between around 0.3 fmol and around 0.6 fmol. Analogous calculations are performed to optimise the amount of library necessary to obtain optimal duplicate rates when using target genomic enrichment, by accounting for the size of the target region.

According to a further aspect of the invention, there is provided a method for detecting mutations present in single DNA molecules, said method comprising the steps of:

(i) generating a nucleic acid library using the method described herein;
(ii) sequencing the nucleic acid library to generate sequencing data; and
(iii) computational analysis of the sequencing data of step (ii) to detect the presence and/or identity of a mutation in single DNA molecules.

Computational Analysis

In one embodiment, the computational analysis comprises aligning reads of the sequencing data to a reference genome. It will be appreciated that the reference genome to which the sequencing data is aligned will be selected based on the nucleic acid composition and source of said nucleic acid composition used in the present method. For example, wherein the nucleic acid composition is obtained from a human cell, tissue or population of cells, the reference genome will be a human reference genome, such as the hs37d5 reference genome. Alternatively, wherein the nucleic acid composition is obtained from a non-human cell, tissue or cell population, the reference genome will be a non-human mammalian reference genome, such as a mouse reference genome, other mammalian or non-mammalian reference genome. After alignment, the barcode sequences are recorded as a separate tag in the resulting BAM file to allow the unique identification of sequencing reads originating from either of the strands of the nucleic acid composition and/or the identification of duplicate reads originating from PCR duplication during library preparation.

In a further embodiment, the computation analysis additionally comprises grouping reads of the sequencing data using a combination of fragmentation breakpoint positions, duplex barcode sequences, sequencing read and strand identity. Thus, in some embodiments, the computational analysis comprises identifying and grouping reads of the sequencing data based on the position in the reference genome to which they correspond and/or the nucleic acid fragment from which they derive, such as the single nucleic acid molecule/strand from which they derive.

As will be appreciated, the above embodiments of aligning reads of the sequencing data to a reference genome and grouping/identifying said reads may be performed in any order. For example, in one embodiment, the computational analysis comprises aligning reads of the sequencing data to a reference genome followed by grouping said reads using a combination of fragmentation breakpoint positions, duplex barcode sequences, sequencing read and strand identity. In an alternative embodiment, the computational analysis comprises grouping reads of the sequencing data using a combination of fragmentation breakpoint positions, duplex barcode sequences, sequencing read and strand identity followed by aligning said reads to a reference genome. In another embodiment, the computational analysis comprises aligning reads of the sequencing data to a reference genome followed by identifying and grouping said reads based on the position in the reference genome to which they correspond and/or the nucleic acid fragment from which they derive, such as the single nucleic acid molecule/strand from which they derive. In a further other embodiment, the computational analysis comprises identifying and grouping reads of the sequencing data based on the position in the reference genome to which they correspond and/or the nucleic acid fragment from which they derive, such as the single nucleic acid molecule/strand from which they derive followed by aligning said reads to a reference genome.

In a yet further embodiment, the computational analysis additionally comprises providing a consensus base call quality score. Such consensus base call quality score may be provided using Bayes' theorem to compute the posterior probability of each base call $B$ given the pileup of reads $D$ from one strand of a template molecule at one genomic position. At each position there are four possible genotypes $i \in \{A, C, G, T\}$. The posterior probability may be calculated using:

$$P(B|D) = \frac{P(B)P(D|B)}{\sum_i P(B_i)P(D|B_i)}$$

Under a uniform prior, where any of the four possible genotypes are equally likely, the equation can be simplified to:

$$P(B|D) = \frac{P(D|B)}{\sum_i P(D|B_i)}$$

To calculate $P(D|B)$, information is integrated from reads in D, where $b_j \in \{A, C, G, T\}$ is the base of read $j = 1...d$:

$$P(D|B_i) = \prod_{j=1}^{j=d} P(b_j|B_i)$$

To calculate $P(b_j|B_i)$ the probability that base $b_j$ is an error may be used, calculated from its Phred quality score $q_j$:

$$P(b_j|G_i) = 1 - e_j \; if \; b_j = B_i, otherwise \; e_j/3$$

where

$$e_j = 10^{\frac{-q_j}{10}}$$

It should be noted that the final probability $P(D|B)$ is the probability that the base call is correct after sequencing and not the probability that the base represents the correct genotype of the original template strand, where independence between observations cannot be assumed. $P(B|D)$ may be rescaled into a Phred quality score $Q$ using:

$$Q = -10 \; log_{10} P(B|D)$$

In cases where the two read mates overlap, the consensus base quality may be calculated using both forward and reverse reads.

Thus, in some embodiments, the consensus base call quality score is provided using a Bayesian formula. However, it will be appreciated that any suitable method may be used to derive a consensus base call quality score, for example, using read counts.

In a still further embodiment, the computational analysis additionally comprises filtering false positive base calls at both reference and mutated positions. Such filtering provides a reduced rate of false positive calls. In order to allow direct calculation of mutation rates, it is important to apply the same filtering to call reference and mutated bases. Germline single nucleotide polymorphisms (SNPs) are filtered using a matched normal and an additional mask to filter sites that are problematic may also be used. The matched normal may be obtained by standard protocols known in the art or by sequencing undiluted NanoSeq libraries where sequencing coverage is concentrated. Filtering according to this embodiment comprises one or more (e.g. all) of the following filters:

1. Each read bundle (i.e. group of PCR duplicates) is required to compose at least two reads from each of the two original DNA strands;
2. The consensus base quality score should be at least 60, to guarantee that there is strong support for a given base call from the duplicate reads that form a read bundle;
3. The minimum difference between the primary alignment score (AS) and the secondary alignment score (XS) should be higher than 50, to keep only read pairs with unambiguous mapping. This filter is essential to remove mapping artefacts and a minimum AS-XS of 50 is applied also to the matched normal. For sites where the two mates overlap the minimum of the average AS-XS for forward and reverse mates is taken;
4. The average number of mismatches in a group of reads (forward or reverse) should not be higher than 2. This filter is important to exclude reads with unreliable mappings. In case a consensus base call is different from the reference, mismatches from this call are not considered to calculate the number of mismatches, hence avoiding a bias in the filtering of mutation and reference calls. This filter is also applied to the matched normal. For sites where the two mates overlap the maximum of the average NM for forward and reverse mates was taken;
5. No 5' clips are allowed;
6. No improper pairs are allowed in the read bundle to avoid unreliable mappings;
7. Base calls in read ends, defined as those within 8bps from the 5' or 3' ends, are discarded because these regions are more likely to be unreliably mapped, especially when there are nearby indels;
8. Reads in the read bundle must contain no indels;
9. The matched normal must have at least certain coverage at a given site to make sure a potential SNP would be detected, e.g. at least 15 reads. For standard matched normals it is also required that there are at least 5 reads from each strand;
10. When a mutation is to be called, it is required that the base is not seen with frequency higher than 0.01 in the matched normal; and
11. A site should not overlap the common SNP and noisy sites masks. Base calls failing this requirement are also counted to obtain a qualitative diagnostic of potential contamination. In the presence of contamination, mutation rates increase largely when these masks are not applied.

In one embodiment, filtering comprises all of the above filters. In an alternative embodiment, filtering comprises filters 1-4, 6-7 and 9-11, i.e. filters 5 and 8 are excluded.

Thus, according to one aspect of the invention, there is provided a method of computational analysis of duplex sequencing data, comprising the steps of:

(a) aligning to a reference genome;
(b) grouping reads of the sequencing data using a combination of fragmentation breakpoint positions, duplex barcode sequences, sequencing read and strand identity, or identifying and grouping reads of the sequencing data based on the position in the reference genome to which they correspond and/or the nucleic acid fragment from which they derive;
(c) providing a consensus base call quality score; and
(d) filtering false positive base calls at both reference and mutated positions.

In one embodiment, the order of steps (a) and (b) are reversed.

In another embodiment, the computational analysis comprises extracting a barcode sequence and clipping the remaining sequence provided by barcoded duplex sequencing adaptors. As described hereinbefore, the nucleic acid molecules within the library may comprise the sequence NNNTCA or NNNXTCA when the restriction endonuclease HpyCH4V has been used to fragment the nucleic acid composition (HpyCH4V cuts at the sequence TGCA), where NNN represents a random 3 nucleotide barcode (e.g. a 3 nucleotide tag), T is the adaptor overhang and X is a 'spacer' nucleotide designed to increase nucleotide diversity during sequencing. Thus, in one embodiment, extracting a barcode sequence comprises extracting a 3 nucleotide barcode. In a further embodiment, clipping the remaining sequence provided by barcoded duplex sequencing adaptors comprises clipping the remaining 3 (e.g. TCA) or 4 (e.g. XTCA) nucleotides.

Thus, according to another aspect of the invention, there is provided a method of computational analysis of duplex sequencing data, comprising the steps of:

(a) extracting a barcode sequence and clipping the remaining sequence provided by barcoded duplex sequencing adaptors;
(b) aligning to a reference genome and appending barcode sequence to alignments;
(c) providing a consensus base call quality score; and
(d) filtering false positive base calls at both reference and mutated positions.

In one embodiment, the computational analysis comprises the detection of substitution imbalances. In order to detect asymmetries in substitution patterns, variants are assigned to the forward or reverse strand according to their distance from fragmentation breakpoints. Variants closest to the 5' of the forward read are assigned to the forward strand. Variants closest to the 5' of the reverse read are assigned to the reverse strand and reverse complemented. Variants equidistant from both fragmentation breakpoints are not counted.

In a further embodiment, the computational analysis comprises indel calling. In order to call indels read bundles with potential indels are first identified, defined as those containing sites with at least 90% of forward and reverse reads having an indel. Read bundles with alignment score-secondary alignment score (AS-XS) $\leq$ 50, 5' clipping or with coverage in the matched normal lower than 16 are filtered out. Indels close to read ends (10bp) are not taken into account. For each of the read bundles potentially containing an indel, the corresponding reads are extracted from the sequencing data file, removing PCR duplicate flags and creating a mini read bundle data file. For each of the read bundle data files samtools mpileup are run to generate genotype likelihoods in BCF format, as follows:

samtools mpileup --no-BAQ -d 250 -m 2 -F 0.5 -r $chr:$start-$end --BCF --output-tags DP,DV,DP4,SP -f $ref_genome -o genotype_likelihods.bcf read_bundle.bam where $chr, $start and $end are the mapping coordinates of the read bundle. Next, indels are called and the output normalised using bcftools as follows:

bcftools index -f genotype_likelihods.bcf genotype_likelihods.indexed.bcf

bcftools call --skip-variants snps --multiallelic-caller --variants-only -O v genotype_likelihods.bcf -o bcftools.tmp.vcf

bcftools norm -f $ref_genome bcftools.tmp.vcf > bcftools.tmp2.vcf

For each of the sites involved in an indel it is checked whether it overlaps a site masked by a common SNP and noise masks, in which case the indel is flagged as MASKED and not further analysed.

The final step involves revisiting the matched normal to inspect if there are indels in a window of +/- 5bps around each candidate indel. For this step the bam2R function from R package *deepSNV* may be used. Reads with mapping quality lower than 10 or with any of the following flags are ignored: "read unmapped", "not primary alignment", "read fails platform/vendor quality checks", "read is PCR or optical duplicate" and "supplementary alignment". If the proportion of indels in the matched normal within the +/- 5bp window around the candidate somatic indel is higher than 1%, the indel is disregarded.

In a yet further embodiment, the computational analysis comprises genome masking. Two masks may be used to filter duplex sequencing data. First, common SNPs, defined as those SNPs with allele frequency (AF) > 0.1% and "PASS" flag in gnomAD, or in 1000G in the case of the Y- and mitochondrial chromosomes (n = 27,204,965) are masked. This SNP mask is important to reduce the impact of eventual contaminant human DNA. The second mask is aimed at removing unreliable calls or sites prone to artefacts. To build this noise mask gnomAD indel calls with AF>1% and SNP calls with AF>0.1% which were not flagged as "PASS" were gathered together. The noise mask also contains sites with elevated error-rates. For each genomic position, error-rates are estimated using the fraction of mismatched bases across a panel of 448 sequenced samples. Sites with error-rates >0.01 are incorporated into the noise mask, which finally contains 22,474,160 bps.

In a further embodiment, the computational analysis comprises *in silico* decontamination of nucleic acid from other sources other than the intended input nucleic acid composition. For example, duplex sequencing libraries may be contaminated with DNA from other individuals, cells, tissues or cell populations, such as other human individuals or cells, tissues or cell populations, which may artificially inflate mutation burden estimates, mainly because germline SNPs in the contaminant DNA may appear as somatic mutations. Even a small percentage of contamination can have a large impact on burden estimates. The burden associated to SNPs in the contaminant would be:

$$Burden_{SNP} = \frac{N_{SNP} * f_{cont}}{G}$$

being $N_{SNP}$ the number of SNPs in the contaminant not shared with the sample at hand, $f_{cont}$ the contamination fraction and G the size of the diploid human genome. Accordingly, 1% contamination would result in a $Burden_{SNP}$ of ~$5\times10^{-6}$ if there are 3 million non-shared SNPs. This burden is much higher than the usually observed somatic mutation rates. First, the number of SNPs across 2,504 individuals from the 1000 Genomes Project remaining after filtering with the common SNPs mask was analysed (n=26,111,286). Results show that on average 55,685 SNPs would remain unfiltered for a given contaminant individual. Hence, for 1% contamination, filtering of common SNPs would reduce $Burden_{SNP}$ from $5\times10^{-6}$ to $9\times10^{-8}$ SNPs/bp. The number of unfiltered SNPs varies largely across continental groups, with averages of 25,666 and 82,765 per individual in Europe and South Asia, respectively. Therefore, 1% of contamination may still be problematic. VerifyBamID is a tool routinely used to estimate human contamination from sequencing data. The most recent version is ancestry aware and has been tested for contamination levels above 1%. Presented herein is a simulation which evaluates VerifyBamID performance at 0.1, 0.25, 0.5, 0.75, 1, 2 and 3% contamination levels in nucleic acid libraries prepared using the method described herein. **Figure 4** shows the contamination of two nucleic acid libraries prepared using the method described herein, one generated using a nucleic acid composition obtained from smooth muscle and one generated using a nucleic acid composition obtained from granulocyte sample. Each of the samples was contaminated with the other and results support the ability of VerifyBamID to detect levels of as low as 0.1% (**Figure 4**).

In certain embodiments, the nucleic acid composition is a DNA composition. In a further embodiment, the nucleic acid composition is nuclear DNA. In a yet further embodiment, the nucleic acid composition is the total DNA from a cell or sample, including, for example, nuclear and mitochondrial DNA.

In one embodiment, the nucleic acid composition is obtained from a tissue or population of cells. In a further embodiment, the nucleic acid composition is obtained from a human cell, tissue or population of cells. In a yet further embodiment, the nucleic acid composition is obtained from a non-human mammalian cell, tissue or population of cells, such as a mouse cell, tissue or population of cells or a non-human primate cell, tissue or population of cells. In a still further embodiment, the nucleic acid composition is obtained from a non-mammalian cell, tissue or population of cells, such as a cell, tissue or population of cells from a fish. In one embodiment, the cell, tissue or cell population is a somatic cell, tissue or cell population. In some embodiments, the cell, tissue or population of cells is from a cancer and/or tumour. Thus, in one embodiment, the cell, tissue or cell population is a cancer/tumour cell, tissue or cell population. In an alternative embodiment, the cell, tissue or population of cells is a healthy (e.g. non-cancerous) cell, tissue or cell population.

Therapeutic Methods, Diagnostic Methods and Uses

According to a further aspect of the invention, there is provided a method for detecting mutations present in single DNA molecules from a population of cells, comprising performing a method as defined herein. In one embodiment the population of cells is a polyclonal population of cells. In a further embodiment, the population of cells is a diseased population of cells, such as a population of cancer cells. In another embodiment, the population of cells is a tissue biopsy or a population of cells from a non-cancer disease. In an alternative embodiment, the population of cells is a healthy population of cells, such as a non-cancerous population of cells. In a yet further embodiment, the population of cells is an aged population of cells.

Thus, according to one aspect of the invention, there is provided a method of diagnosing disease in a human or animal subject, said method comprising the steps of:

(i) detecting the presence of a mutation in single DNA molecules according to the methods defined herein; and
(ii) using the presence and/or identity of the mutation as an indicator of disease in the human or animal subject.

According to a further aspect of the invention, there is provided a method for the assessment of a human or animal subject for suitability for medical treatment, said method comprising the steps of:

(i) detecting the presence of a mutation in single DNA molecules according to the methods defined herein; and
(ii) using the presence and/or identity of the mutation to determine the selection of a suitable treatment for the human or animal subject.

The methods described herein find particular utility in identifying driver mutations, such as disease driver mutations, in highly polyclonal samples. For example, whole-exome nanorate sequencing (NanoSeq) using methods described herein in the context of polyclonal disease maximises the ability to discover genes affected by mutations in different clones by combining: (i) NanoSeq accuracy (typically $<5\times10^{-9}$) to detect mutations in single molecules of DNA, (ii) whole-exome coverage (e.g. using sonication to fragment the nucleic acid composition, to enable the discovery of driver mutations not previously known to be involved in disease), and (iii) highly polyclonal samples. In particular, it will be appreciated that in a more polyclonal sample the chances of sampling a different clone with each molecule are maximised. Thus, the methods described herein provide accurate identification of mutations in single molecules of DNA which allows the ability to identify driver mutations down to single DNA molecules within a polyclonal sample.

By way of specific example, using previous sequencing methods with error rates higher than the typical somatic mutation rates of human cells, mutations can only be accurately be 'called' when they are found in more than 1 mutant molecules. Therefore, if a sample of blood (containing millions of cells) is sequenced using 1,000x sequencing and at least 2 molecules are needed to for a mutation to be identified, mutations can only be detected if they are present in clones making up at least 0.4% of the cells in the blood sample (2/1000 = 0.2%, but cells are diploid and mutations tend to be in one of the two copies, hence 0.4% clones may appear in approx. 2 reads in 1,000x duplex sequencing). That is, requiring 2 or more mutant molecules supporting a mutation limits sensitivity to mutations present in at least a fraction of a percent of cells in a sample. If 20% of cells in the blood sample have acquired a pathogenic mutation during life but these mutations are present only in single cells or small clones (e.g. no mutation is present in more than 1,000 cells, so no mutation reaches 0.1% frequency in the sample of millions of cells), then sequencing methods requiring 2 or more molecules to detect a mutation would not identify any pathogenic or driver mutations in the sample. In contrast, if NanoSeq as described herein is used (which allows the accurate identification of mutations in single DNA molecules), then approx. 100 pathogenic/driver mutations would be found in the sample (1,000x duplex sequencing will effectively sample 500 diploid cell equivalents, of which 20% have a pathogenic mutation in this example). This is the difference that is achieved when using single-molecule sensitivity in the context of highly polyclonal samples. As such, the NanoSeq methods described herein provide the ability to perform DNA sequencing with accurate single-molecule calling with error rates $<5\times10^{-9}$ and with coverage across the whole genome for identifying mutation burden and mutation signatures or across the whole exome or large gene panels for driver mutation discovery. In addition, the total percentage of cells in the sample carrying a mutation in a given disease-related gene can be estimated. Whereas previous duplex sequencing methods have been used to detect driver mutations in single molecules of DNA (see e.g. Salk *et al.* (2018) doi: https://dx.doi.org/ 10.2139/ssrn.3307592), we note that higher error rates (typically $>1\times10^{-7}$) limit the accuracy of the mutations detected and that, to our knowledge, these methods have only been applied to single genes or very small panels of genes, preventing the exome-wide discovery of unknown driver genes.

In one embodiment, the mutation in a single DNA molecule is a driver mutation, such as a disease driver mutation. Such driver mutations are associated with and may also be causative of an aged or diseased stage, such as cancer. Thus, in some embodiments the driver mutation is involved in an ageing process or is a disease driver mutation. In one embodiment, the driver mutation is a driver mutation associated with cancer. In another embodiment, the driver mutation is associated with a disease other than cancer. In particular embodiments, the driver mutation is in the exome. Thus, in a further embodiment the driver mutation is in the CDS of a gene, such as in one or more protein coding sequence. In a yet further embodiment, the driver mutation is a mutation of one or more CDS, such as a mutation of one or more protein coding sequence.

In a further aspect of the invention there is provided a method of identifying a driver mutation, such as a disease driver mutation, in a polyclonal sample, said method comprising the steps of:

(i) detecting the presence of a mutation in single DNA molecules according to the methods defined herein; and
(ii) using the presence, identity and/or location of the mutation as an indicator of whether the mutation is a driver mutation.

The presence, identity and/or location of the mutation can be useful in determining if the mutation is a driver mutation, for example, by identifying the gene in which said mutation is present and/or whether the mutation will lead to a change in the encoded protein sequence. Mutations in protein sequences can lead to altered protein activity (e.g. increased or decreased activity), cellular localisation, secretion, cellular accumulation and the like. Examples of driver mutations in non-coding genomic regions include those which alter the expression, such as the expression level, of coding sequences.

In certain embodiments, the disease is cancer or the treatment is for the treatment of cancer. For example, in one embodiment, the method of diagnosing disease in a human or animal subject described herein is for monitoring relapse, such as cancer relapse, in a biopsy from said human or animal subject, such as a liquid biopsy. Thus, in one embodiment, the treatment is an anti-cancer treatment, such as an anti-cancer therapy. Suitable anti-cancer treatments include surgery, radiotherapy, chemotherapy, immunotherapy, hormone therapy and biological therapy. It will be appreciated that the specific selection of a suitable anti-cancer treatment will depend on the type, location (e.g. the tissue from which the cancer originates) and stage of the particular cancer to be treated. For example, the suitability of the human or animal subject for treatment may depend on the type, location and stage of cancer. Therefore, it will be appreciated that the methods defined herein provide information for the presence and/or identity of the disease, such as the presence and/or identity of a cancer, by providing the presence and/or identity of a mutation in single nucleic acid molecules, such as single DNA molecules. In a further embodiment, the driver mutation is a cancer driver mutation, such as a mutation in an oncogene or a tumour suppressor gene. In alternative embodiments, the disease is a disease other than cancer. Thus, in one embodiment the driver mutation is a mutation in a gene other than an oncogene or a tumour suppressor gene.

According to a yet further aspect of the invention, there is provided an *in vitro* method for screening a suspected mutagenic agent or the mutagenic profile of a mutagenic agent, said method comprising the steps of:

(i) exposing an *in vitro* cell culture to the suspected mutagenic agent or exposing an organism to a mutagenic agent, such as a chemotherapeutic agent;
(ii) detecting the presence and/or identity of a mutation in single DNA molecules in the cell culture or in non-invasive liquid or solid tissue samples obtained from the organism, according to the methods defined herein; and
(iii) using the presence and/or identity of the mutation to determine the mutagenic potential of the suspected mutagenic agent or the mutagenic profile of the mutagenic agent.

In one embodiment, the organism exposed to a mutagenic agent is a human, such as a human subject undergoing chemotherapy. In another embodiment, the organism is a non-human mammal, such as a mouse or a non-human primate. In a further embodiment, the organism is a non-mammalian animal, such as a fish or nematode worm, e.g. *C. elegans.* In a yet further embodiment, the *in vitro* cell culture is a culture of cancer and/or tumour cells, such as cells from a cancer and/or tumour. In an alternative embodiment, the *in vitro* cell culture is a culture of healthy (e.g. non-cancerous) cells, such as from a healthy tissue.

In some embodiments, the mutagenic agent is a chemotherapeutic agent. In further embodiments, the mutagenic agent is a radiotherapeutic agent. In a yet further embodiment, the mutagenic agent is a mutagenic process, such as gene editing.

In a further aspect of the invention, there is provided an *in vitro* method for detecting the presence and/or identity of a suspected mutation in a cell, said method comprising the steps of:

(i) culturing a cell *in vitro* and/or subjecting an *in vitro* cell culture to a mutagenic process, such as by performing gene editing; and
(ii) detecting the presence and/or identity of a mutation in a single DNA molecule in the cell or cell culture, according to the methods described herein.

In one embodiment, the suspected mutation in a cell is caused by *in vitro* culture. Such *in vitro* culture may include the growth of cells in culture/*in vitro,* maturation of cells in culture, differentiation of stem-like cells, such as induced pluripotent stem cells (iPSCs), and the generation of organoids. In a further embodiment, the suspected mutation is caused by a mutagenic process, such as gene editing. Methods of gene editing are well known in the art and may include editing by CRISPR-Cas9, TALENS and the like. Other mutagenic processes include radiation. In a particular embodiment, the suspected mutation is caused by a combination of *in vitro* culture and a mutagenic process, e.g. gene editing. Thus, in a further embodiment the suspected mutation is a genetic edit. In a yet further embodiment, the suspected mutation is an off-target mutation.

The following studies and protocols illustrate embodiments of the methods described herein and their suitability for use:

## EXAMPLES

Example 1 - Applying NanoSeq to a 59-year-old Sample of Granulocytes

In order to showcase the methods defined herein and determine their suitability for duplex sequencing, terminally differentiated granulocytes for an 59-year old male donor for which 110 blood colonies were available were analysed by NanoSeq. The data presented in **Figure 1** demonstrates that NanoSeq provides very similar mutation burden estimates between granulocytes and single cell-derived blood colonies compared to BotSeqS which provides a much higher burden estimate (see **Figure 1C**). Furthermore, while standard BotSeqS protocols produced data with high substitution imbalances, these were absent from NanoSeq data (see **Figure 1E**).

Therefore, these data show the ability of NanoSeq to provide an accurate detection and identification of mutations in a sample of terminally differentiated somatic cells.

Example 2 - The Error Rate of NanoSeq is Lower than 5 Errors per Billion Sites

To quantify the error rate of NanoSeq, the method was applied to samples with very low mutation burden. Sperm from a 21-year-old donor was chosen due to the low mutation rate of the germline, as well as cord blood granulocytes from two neonates. Seven replicates of the sperm sample yielded burden estimates consistent with current estimates of the mutation rate in the parental germline from trio studies (Kong *et al.* (2012) doi: https://doi.org/10.1038/nature11396 and Rahbari *et al.* (2016) dori: https://doi.org/10.1038/ng.3469), with ~52 mutations per haploid sperm cell ($1.8 \times 10^{-8}$ mutations/bp or ~2.5 mutations/year/cell) **(Figure 1F)**. NanoSeq estimates from cord blood granulocytes were compared to 100 single cell-derived cord blood colonies from two different donors. Corrected NanoSeq estimates were higher than blood colonies (109 vs 66 mutations per cell; 95% Poisson confidence intervals 95-125; **Figure 1G**). This difference could be due to NanoSeq errors, higher burden in differentiated granulocytes than stem-cell-derived colonies, or both. Comparing substitution profiles can help address this. A bootstrapping approach was used to estimate the cosine similarity expected between the NanoSeq and the colony mutational spectra if they were identical, given the number of mutations available. This revealed an indistinguishable spectrum, consistent with most mutations detected by NanoSeq being genuine. Together, the sperm and cord blood data indicate that the error rate of NanoSeq is considerably lower than $5 \times 10^{-9}$ errors/bp, approximately two orders of magnitude lower than the BotSeqS error rate and the somatic mutation burden of most human tissues studied to date. Analysis of insertions and deletions (indels) in cord blood similarly confirms that the NanoSeq indel error rate is $<3 \times 10^{-9}$ errors/bp. These error rates open the door to the study of mutagenesis in any tissue type.

Despite enabling low error rates and reducing DNA input requirements, a disadvantage of using restriction enzymes is that their sequence specificity gives partial coverage of the genome (e.g. 28.7% using HpyCHV4). While the covered genome is sufficiently random to enable accurate and unbiased estimation of mutation rates and signatures, some applications such as targeted sequencing would benefit from full genome coverage. Alternative approaches for clean random fragmentation or error-free end-repair, such as exonuclease blunting after sonication, or the use of multiple restriction enzymes as described hereinbefore may therefore be utilised.

Example 3 - Mutations in Blood and Colonic Stem Cells Dominate the Burden in Differentiated Cells

Most knowledge on somatic mutation in normal tissues is restricted to mutagenesis in stem or proliferating cells, owing to the reliance of current approaches on *in vivo* or *in vitro* clones. Stem cells are assumed to be genetically more stable than differentiated cells, and stem cell hierarchies have been proposed to protect adult tissues from the accumulation of somatic mutations (Cairns J (1975) doi: https://doi.org/10.1038/255197a0). This raises the possibility that differentiated cells may show considerably higher rates of somatic mutation, but quantifying this has remained challenging (Brazhnik *et al.* (2020) doi: https://doi.org/10.1126/ sciadv.aax2659).

This question was first addressed in the haematopoietic system, using NanoSeq to compare the mutational landscape of mature granulocytes to that of immature haematopoietic stem or progenitor cells (HSPCs). Samples of granulocytes from peripheral blood from healthy donors were duplex sequenced followed by standard whole-genome sequencing in single cell-derived colonies from HSPCs. A previous study reported that long-term HSCs and multipotent progenitor cells show similar mutation rates (Orsorio *et al.* (2018) doi: https://doi.org/10.1016/ j.celrep.2018.11.014). Results presented in **Figure 5** show that terminally differentiated granulocytes have very similar mutation burden and mutational signatures to HSPCs (see **Figure 5A**). This is intriguing given that estimates from mice would suggest that, on average, terminally differentiated blood cells have undergone considerably more divisions than HSPCs (MacKey MC (2001) doi: https://doi.org/10.1046/j.1365-2184.2001.00195.x and Catlin *et al.* (2011) doi: https://doi.org/10.1182/blood-2010-08-303537).

It can be argued that HSPCs are a heterogeneous population and that estimates of mutation burden from HSPCs successfully grown *in vitro* may not be reflective of the mutation rate in true slow-cycling stem cells responsible for long-term maintenance of the haematopoietic system. However, an alternative estimate of the somatic mutation rate in effective long-term HSCs is available from the slope of the linear increase of somatic mutations with age in both colonies and granulocytes (~20.6 mutations/year, $CI_{95\%}$: 15.8-25.2). If cells accumulated considerable numbers of mutations after leaving the long-term stem cell compartment, this should manifest as a large intercept in the regression model. Instead, the strong linear relationship with a very small intercept suggests that the vast majority of the mutations in adult granulocytes accumulate in long-term stem cells, and that only a small minority of mutations are accrued during transient proliferation and terminal differentiation.

A similar analysis was then performed on colonic epithelium. Estimates of the somatic mutation rate in colonic stem cells are available from whole-genome sequencing of clonal organoids derived from Lgr5+ cells and from sequencing of single colonic crypts (Lee-Six et al. (2019)). Genome sequencing of whole crypts can be used to estimate the somatic mutation rate of colonic stem cells, as colonic crypts are clonally derived from a single stem cell. However, the process of reaching clonality through drift in the population of stem cells in a crypt is estimated to take several years in humans (Nicholson *et al.* (2018) doi: https://doi.org/10.1016/j.stem.2018.04.020), a process that could conceivably explain some of the heterogeneity in mutation burden observed in crypts within donors (Lee-Six *et al.* (2019)). NanoSeq was applied to laser microdissected colonic crypts to measure the mutation burden of differentiated cells in colonic epithelium and without the genetic drift time lag of standard single-crypt sequencing. Mutational signatures from differentiated cells in colonic epithelium were overall consistent with those found by previous studies on colonic stem cells, with a dominance of SBS1, SBS5 and colibactin (**Figure 5B**; Pleguezuelos-Manzano *et al.* (2020) doi: https://doi.org/10.1038/s41586-020-2080-8). Further confirming the quality of the indel calls, the donor with a high number of mutations attributed to the colibactin signature displayed the characteristic indel signature reported for colibactin exposure (**Figure 6**).

Excluding mutations associated with colibactin and focusing on ubiquitous mutational signatures observed across donors, an estimate for the mutation rate of differentiated epithelial cells was obtained and found to be slightly higher but not significantly different to that from stem cells using organoids or laser microdissection (53.8 mutations/year, $CI_{95\%}$: 41.0-65.7; **Figure 5B**). Differences among NanoSeq estimates from different cuts from the same donor suggest that within-donor variability is not due to variable time lags of clonal drift but to mutation rate variability across crypts, consistently with data from clonal organoids.

Overall, NanoSeq data on granulocytes and colonic epithelium yielded similar estimates of mutation burden and mutational signatures to their corresponding stem cells. These results provide an early view into the somatic mutation landscape of two differentiated cell types.

Example 4 - Mutational Landscape of Smooth Muscle, a Highly Polyclonal Tissue

Smooth muscle is a highly polyclonal tissue, not amenable to standard sequencing approaches and whose somatic mutation landscape remains unknown. Smooth muscle from 10 donors and from two different organs, bladder and colon, was collected using laser microdissection. As expected, standard whole-genome sequencing on these cuts revealed few mutations at low allele frequencies. However, using NanoSeq, substitution and indel burdens increased linearly with age, with rates of 24.7 ($CI_{95\%}$: 22.5-27.0) substitutions and 2.1 ($CI_{95\%}$: 1.7-2.5) indels per year (**Figure 7A-7B**). Despite their different anatomical origin, smooth muscle cells from the bladder and colon walls showed similar mutation rates (linear regression, $P = 0.6$), although the indel rate appeared slightly higher in bladder ($P = 0.04$). These burden estimates are higher than reported estimates in skeletal muscle satellite cells grown *in vitro* ($13.1 \pm 2.7$ SNVs per year, Franco *et al.* (2018)).

The mutation spectra were relatively similar to those recovered in granulocytes (**Figure 7C-7D** ,**Figure 1D**). Signature decomposition was then performed on mutation data from smooth muscle (**Figure 7C**), granulocytes, colonic crypts (excluding those with the colibactin signature), and neurons. Three main signatures were extracted (**Figure 7E**). Signature B was a close match to SBS1 (C>T changes at CpG dinucleotides), while signatures A and C imperfectly resemble SBS5 and SBS16, respectively. It is conceivable that SBS5 reflects a collection of co-occurring processes, rather than a single mutational process, leading to some differences across tissues. All three signatures accumulated linearly with age in smooth muscle. The contributions of A, B, and C signatures were also similar in muscle from bladder and colon and across donors (**Figure 7F**). Altogether, data from internal cell types, including granulocytes, smooth muscle and neurons reveals more limited variation in mutation rate across individuals than it has been observed in epithelia exposed to external mutagens, such as skin, colon (**Figure 5B**), bronchus or bladder.

Example 5 - Lifelong Mutagenesis in Post-Mitotic Neurons

Cortical neurons are prime examples of post-mitotic cells, which makes them inaccessible to traditional sequencing methods that require clonality. Single-cell sequencing has provided suggestive insights into somatic mutation in neurons (Lodato *et al.* (2015), Lodato *et al.* (2018) and Bae *et al.* (2018) doi: https://doi.org/10.1126/science.aan8690), although it is unclear to what extent multiple displacement amplification artefacts clouded these results. Despite the technical obstacles, a possible role of somatic mutation in neurodegeneration has attracted considerable interest, with some studies suggesting a link between the two (Park JS *et al.* (2019) doi: https://doi.org/10.1038/s41467-019-11000-7 and Lodato *et al.* (2018)). Furthermore, the general lack of DNA replication in cortical neurons makes them an important cell type to study somatic mutagenesis in the absence of cell division.

NanoSeq was applied to frontal cortex neurons from 8 healthy donors and 9 Alzheimer's disease (AD) patients, using nuclei sorting with the NeuN neuronal marker. These data revealed a tight linear accumulation of 20.0 substitutions (linear model $CI_{95\%}$: 19.1-20.9) and 3.1 indels ($CI_{95\%}$: 2.9-3.3) per year, seemingly constant throughout life (**Figure 7G-7H**). This confirms that mutations accumulate in a clock-like fashion in cortical neurons, in the absence of replication, consistent with observations from single-cell sequencing. Although the difference is small, AD donors showed a significantly lower substitution rate than healthy donors ($P = 0.006$; 19.1 ($CI_{95\%}$: 18.1-20.0) vs 21.6 ($CI_{95\%}$: 20.5-22.7)). The difference could be explained by lower rates of mutations associated to signatures A and B but not C in AD ($p^A = 0.02$;

$p^B$ = 0.03; $p^C$ = 0.55; **Figures 7K** and **8**). Differences in mutation burden between controls and AD donors could be a consequence of the pathogenesis of the disease, including differences in metabolism or different death rates across subpopulations of neurons in AD.

The substitution and indel spectra of neurons (**Figure 7I-7J**) are different to those of granulocytes (**Figure 1D**) and smooth muscle (**Figure 7C**). T>C substitutions are more frequent in neurons, especially at ATN trinucleotides, which manifests as a higher contribution of signature C (**Figure 7K**). Interestingly, despite the absence of DNA replication, signature B (SBS1), which is often linked to cell division, accumulates linearly with age in neurons (1.8 substitutions per year, linear model $CI_{95\%}$: 0.23-3.3, $P$ = 0.03; **Figure 8**). The clear presence of C>T mutations at CpG sites in neurons is better appreciated normalising the rates by the trinucleotide sequence composition in the genome (**Figure 9**), and implies that C>T mutations caused by 5-methylcytosine deamination can be fixed in both DNA strands without replication. In contrast to previous reports (Lodato *et al.* (2015)), a clear association was not found between expression levels and substitution rates across genes (**Figure 7L**).

Indel analysis revealed a higher relative frequency of indels in neurons than in other tissues, caused by an unusual signature characterised by indels longer than 1bp (**Figures 7D, 7J** and **10**). This indel signature and its association with highly expressed genes has some resemblance to a little-understood mutational process recently described in cancer genomes (Rheinbay *et al.* (2020) doi: https://doi.org/10.1038/s41586-020-1965-x; **Figure 11**). While this signature had been speculated to be caused by replication-transcription collisions, a different mechanism must be responsible for it in post-mitotic neurons.

Therefore, the data presented herein demonstrate that NanoSeq, building on duplex sequencing approaches, enables the reliable detection of mutations in single molecules of DNA. One important application of this technology is the ability to study mutagenesis in any cell type, including differentiated and post-mitotic cells. A remarkable observation that emerges from these data is that mutation rates vary modestly across somatic cell types, largely independently of cell division rates (**Figure 7O**). Indeed, similar mutation rates are found in non-dividing cortical neurons, in smooth muscle and in blood; or in colonic epithelium, which divides every few days, and in mostly quiescent hepatocytes or urothelial cells. Although DNA replication and cell division have been assumed to be major sources of mutations, the linear accumulation of somatic mutations in post-mitotic neurons confirms that dominant mutational processes can occur independently of replication. The similar mutation burden and signatures in long-term stem cells and in granulocytes could also be consistent with a time-dependent rather than division-dependent accumulation of somatic mutations during haematopoiesis, although other explanations are possible. Altogether, it is conceivable that replication-independent mutational processes play a larger role in somatic mutagenesis than it is commonly assumed.

Beyond enabling studies on mutagenesis across tissues, the ability to obtain accurate and efficient estimates of average mutation burden and mutational signatures in genetically heterogeneous populations of cells will have wider applications. NanoSeq may be used for *in vitro* mutagenesis studies and screens, using cell cultures exposed to different mutagens, without the need of single-cell bottlenecks after exposure (Kucab *et al.* (2019) doi: https://doi.org/10.1016/j.cell.2019.03.001). Being insensitive to clonality, NanoSeq could also be used to quantify mutation rates in liquid or non-invasive tissue samples, enabling population-scale studies of somatic mutagenesis across conditions and risk factors, in health and disease.

Example 6 - Discovery of Driver Mutations in Non-Cancer Disease Using Exome NanoSeq

Positively-selected driver mutations in key metabolic pathways have recently been reported in the context of chronic liver disease, including non-alcoholic fatty liver disease (NAFL) and non-alcoholic steatohepatitis (NASH). Performing low-input whole-genome sequencing of 1,590 laser microdissections of liver tissue from 34 liver samples, researchers found evidence of convergent somatic mutations in *FOXO1*, *CIDEB* and *GPAM* in some patients (Ng *et al.* (2021)). These mutations affected insulin signalling or the regulation of lipid metabolism and were speculated to contribute to the pathogenesis of chronic liver disease.

Therefore, to exemplify the potential of whole-exome NanoSeq for the discovery of driver mutations in the context of polyclonal disease, DNA was extracted from whole histology sections from several biopsies of liver from one of the patients in that study. These DNA samples were then subject to whole-exome NanoSeq, reaching in aggregate a mean whole-exome single-molecule-resolution coverage of ~100x. These identified 2 hotspot mutations in *FOXO1* and 1 mutation in *CIDEB* (**Table 2**). All three mutations were detected in single molecules of DNA, demonstrating the importance of single molecule sensitivity and the potential of whole-exome NanoSeq applied to large polyclonal areas of diseased tissue for driver discovery. The mutational spectrum and mutation burden estimated from the whole-exome NanoSeq data was also consistent with previous studies of somatic mutations in normal and diseased liver (Blockzijl *et al.* (2016) doi: https://doi.org/10.1038/nature19768; and Brunner et al. (2019) doi: https://doi.org/10.1038/s41586-019-1670-9) (**Figure 12**).

Table 2 - Hotspot mutations identified in liver biopsies using NanoSeq.

| Gene | Mutation | Coverage | Q-value | Statistical test |
|------|----------|----------|---------|-----------------|
| **FOXO1** | S22W chr13:41240285 C>G | 96x | 0.013 | sitednds on 568 cancer genes |
| **FOXO1** | S22W chr13:41240285 C>G | 96x | 0.013 | sitednds on 568 cancer genes |
| **CIDEB** | R45Q chr14:24776629 C>T | 124x | - | mutation in interaction domain (PMID:34646017) |

To estimate the error rate of whole-exome NanoSeq, whole-exome NanoSeq was performed at a single-molecule-resolution coverage of ~100x in a sample of cord blood granulocytes. The resulting estimated mutation burden (~$9 \times 10^{-9}$ mutations/bp when extrapolated into the whole-genome) and the mutation spectrum were both statistically consistent with the known mutation landscape of cord blood stem cells from the sequencing of single-cell derived blood colonies (**Figure 12**). This confirms that the error rate of whole-exome NanoSeq in these data was considerably lower than $5 \times 10^{-9}$ errors/bp, in line with the error rate of the NanoSeq protocol using restriction enzymes.

**Claims**

1. A method of generating a nucleic acid library for sequencing, said method comprising the steps of:

    (i) fragmenting a nucleic acid composition to generate fragmented nucleic acid molecules with blunt ends;
    (ii) introducing dideoxy nucleotides at internal nick sites present in the fragmented nucleic acid molecules with blunt ends and tailing to generate tailed nucleic acid fragments; and
    (iii) adding sequencing adaptors to the tailed nucleic acid fragments to generate a nucleic acid library.

2. The method according to claim 1, wherein fragmenting the nucleic acid composition in step (i) comprises using one or more blunt/non-cohesive end-generating endonuclease,

    optionally wherein using a blunt/non-cohesive end-generating endonuclease prevents the need for end-repair of the fragmented nucleic acid molecules, and/or
    optionally wherein the blunt/non-cohesive end-generating endonuclease is a blunt/non-cohesive end-generating restriction endonuclease having a 4 base-pair recognition site and/or which is not impaired by overlapping CpG methylation,
    optionally wherein the blunt/non-cohesive end-generating restriction endonuclease is selected from one or both of: AluI and HpyCH4, such as HpyCH4III, HpyCH4IV or HpyCH4V.

3. The method according to claim 1, wherein fragmenting the nucleic acid composition in step (i) comprises removing overhangs from the fragmented nucleic acid molecules using one or more exonuclease enzyme to generate fragmented nucleic acid molecules with blunt ends,
optionally wherein fragmenting the nucleic acid composition in step (i) comprises mechanical fragmentation, such as sonication.

4. A method of generating a nucleic acid library for sequencing, said method comprising the steps of:

    (i a) fragmenting a nucleic acid composition by sonication to generate fragmented nucleic acid molecules;
    (i b) removing overhangs from the fragmented nucleic acid molecules using one or more exonuclease enzyme to generate fragmented nucleic acid molecules with blunt ends;
    (ii) introducing dideoxy nucleotides at internal nick sites present in the fragmented nucleic acid molecules and tailing to generate tailed nucleic acid fragments; and
    (iii) adding sequencing adaptors to the tailed nucleic acid fragments to generate a nucleic acid library.

5. The method according to claim 3 or claim 4, wherein the one or more exonuclease enzyme is Mung Bean nuclease.

6. The method according to any one of claims 1 to 5, wherein introducing dideoxy nucleotides at internal nick sites in step (ii) prevents extension of the internal nick and results in an unamplifiable nucleic acid strand, and/or wherein:

(i) the dideoxy nucleotides introduced in step (ii) are dideoxy non-A nucleotides, such as ddBTPs, and optionally the tailing of the fragmented nucleic acid molecules in step (ii) comprises A-tailing; or
(ii) the dideoxy nucleotides introduced in step (ii) are dideoxy non-C nucleotides, such as ddDTP, and optionally the tailing of the fragmented nucleic acid molecules in step (ii) comprises C-tailing; or
(iii) the dideoxy nucleotides introduced in step (ii) are dideoxy non-G nucleotides, such as ddHTP, and optionally the tailing of the fragmented nucleic acid molecules in step (ii) comprises G-tailing; or
(iv) the dideoxy nucleotides introduced in step (ii) are dideoxy non-T nucleotides, such as ddVTP, and optionally the tailing of the fragmented nucleic acid molecules in step (ii) comprises T-tailing.

7. The method according to any one of claims 1 to 6, wherein the sequencing adaptors are duplex sequencing adaptors comprising a barcode sequence.

8. The method according to any one of claims 1 to 7, wherein the nucleic acid library is for duplex sequencing.

9. The method according to claim 8, wherein the nucleic acid library is diluted to a concentration which maximises duplex coverage as predicted using an analytical and empirical model,
optionally wherein the duplex coverage is between around 15x and 30x whole-genome equivalent and the nucleic acid library is diluted to between around 0.3 fmol and around 0.6 fmol.

10. The method according to any one of claims 1 to 9, additionally comprising the step of:

(iv) selectively enriching nucleic acids corresponding to one or more genomic region of interest to generate an enriched nucleic acid library,
optionally wherein the one or more genomic region of interest is one or more coding sequences (CDSs) of coding genes, such as protein coding sequences, and optionally wherein the method is for generating a nucleic acid library for exome sequencing and selectively enriching nucleic acids corresponding to genomic regions of interest in step (iv) comprises using oligonucleotide probes which bind to the coding sequences (CDSs).

11. A method for detecting mutations present in single DNA molecules, said method comprising the steps of:

(i) generating a nucleic acid library or an enriched nucleic acid library using the method according to any one of claims 1 to 10;
(ii) sequencing the nucleic acid library or the enriched nucleic acid library to generate sequencing data; and
(iii) computational analysis of the sequencing data of step (ii) to detect the presence and/or identity of a mutation in single DNA molecules,

optionally wherein the computational analysis of step (iii) comprises:

(a) aligning to a reference genome;
(b) grouping reads of the sequencing data using a combination of fragmentation breakpoint positions, duplex barcode sequences, sequencing read and strand identity, or identifying and grouping reads of the sequencing data based on the position in the reference genome to which they correspond and/or the nucleic acid fragment from which they derive;
(c) providing a consensus base call quality score; and
(d) filtering false positive base calls at both reference and mutated positions.

12. A method for detecting a mutation present in a single DNA molecule from a population of cells, comprising performing the method according to any one of claims 1 to 11,

optionally wherein the population of cells is a polyclonal population of cells, and/or
optionally wherein the population of cells is an aged population of cells or a diseased population of cells, and/or
optionally wherein the mutation present in a single DNA molecule is a driver mutation.

13. The method according to claim 12, wherein the driver mutation is involved in an ageing process or is a disease driver mutation.

14. A method of diagnosing disease in a human or animal subject, said method comprising the steps of:

(i) detecting the presence and/or identity of a mutation in a single DNA molecule according to the method of claim 12 or claim 13; and
(ii) using the presence and/or identity of the mutation as an indicator of disease in the human or animal subject.

15. An *in vitro* method for detecting the presence and/or identity of a suspected mutation in a cell, said method comprising the steps of:

(i) culturing a cell *in vitro* and/or subjecting an *in vitro* cell culture to a mutagenic process, such as by performing gene editing; and
(ii) detecting the presence and/or identity of a mutation in a single DNA molecule in the cell or cell culture, according to the method of claim 12 or claim 13.

**Patentansprüche**

1. Verfahren zum Erzeugen einer Nukleinsäurebibliothek zur Sequenzierung, wobei das Verfahren die folgenden Schritte umfasst:

(i) Fragmentieren einer Nukleinsäurezusammensetzung, um fragmentierte Nukleinsäuremoleküle mit stumpfen Enden zu erzeugen;
(ii) Einführen von Didesoxynukleotiden an internen Nick-Stellen, die in den fragmentierten Nukleinsäuremolekülen mit stumpfen Enden vorhanden sind, und Schwanzbildung, um geschwänzte Nukleinsäurefragmente zu erzeugen; und
(iii) Hinzufügen von Sequenzierungsadaptern zu den geschwänzten Nukleinsäurefragmenten, um eine Nukleinsäurebibliothek zu erzeugen.

2. Verfahren nach Anspruch 1, wobei das Fragmentieren der Nukleinsäurezusammensetzung in Schritt (i) das Verwenden einer oder mehrerer stumpfer/nichtkohäsiver enderzeugender Endonukleasen umfasst,

optional wobei das Verwenden einer stumpfen/nichtkohäsiven enderzeugenden Endonuklease die Notwendigkeit einer Endreparatur der fragmentierten Nukleinsäuremoleküle verhindert und/oder
optional wobei die stumpfe/nichtkohäsive enderzeugende Endonuklease eine stumpfe/nichtkohäsive enderzeugende Restriktionsendonuklease mit einer 4-Basenpaar-Erkennungsstelle ist und/oder nicht durch überlappende CpG-Methylierung beeinträchtigt wird,
optional wobei die stumpfe/nichtkohäsive enderzeugende Restriktionsendonuklease ausgewählt ist aus einem oder beiden von: Alul und HpyCH4, wie etwa HpyCH4III, HpyCH4IV oder HpyCH4V.

3. Verfahren nach Anspruch 1, wobei das Fragmentieren der Nukleinsäurezusammensetzung in Schritt (i) das Entfernen von Überhängen aus den fragmentierten Nukleinsäuremolekülen unter Verwendung eines oder mehrerer Exonuklease-Enzyme umfasst, um fragmentierte Nukleinsäuremoleküle mit stumpfen Enden zu erzeugen, optional wobei das Fragmentieren der Nukleinsäurezusammensetzung in Schritt (i) eine mechanische Fragmentierung, wie etwa Beschallung, umfasst.

4. Verfahren zum Erzeugen einer Nukleinsäurebibliothek zur Sequenzierung, wobei das Verfahren die folgenden Schritte umfasst:

(ia) Fragmentieren einer Nukleinsäurezusammensetzung durch Beschallung, um fragmentierte Nukleinsäuremoleküle zu erzeugen;
(ib) Entfernen von Überhängen aus den fragmentierten Nukleinsäuremolekülen unter Verwendung eines oder mehrerer Exonuklease-Enzyme, um fragmentierte Nukleinsäuremoleküle mit stumpfen Enden zu erzeugen;
(ii) Einführen von Didesoxynukleotiden an internen Nick-Stellen, die in den fragmentierten Nukleinsäuremolekülen vorhanden sind, und Schwanzbildung, um geschwänzte Nukleinsäurefragmente zu erzeugen; und
(iii) Hinzufügen von Sequenzierungsadaptern zu den geschwänzten Nukleinsäurefragmenten, um eine Nukleinsäurebibliothek zu erzeugen.

**5.** Verfahren nach Anspruch 3 oder Anspruch 4, wobei das eine oder die mehreren Exonuklease-Enzyme Mungbohnen-Nuklease ist.

**6.** Verfahren nach einem der Ansprüche 1 bis 5, wobei das Einführen von Didesoxynukleotiden an internen Nick-Stellen in Schritt (ii) eine Extension des internen Nick verhindert und zu einem nicht amplifizierbaren Nukleinsäurestrang führt und/oder wobei:

(i) die in Schritt (ii) eingeführten Didesoxynukleotide Didesoxy-Nicht-A-Nukleotide, wie etwa ddBTP, sind und optional die Schwanzbildung der fragmentierten Nukleinsäuremoleküle in Schritt (ii) A-Schwanzbildung umfasst; oder

(ii) die in Schritt (ii) eingeführten Didesoxynukleotide Didesoxy-Nicht-C-Nukleotide, wie etwa ddDTP, sind und optional die Schwanzbildung der fragmentierten Nukleinsäuremoleküle in Schritt (ii) C-Schwanzbildung umfasst; oder

(iii) die in Schritt (ii) eingeführten Didesoxynukleotide Didesoxy-Nicht-G-Nukleotide, wie etwa ddHTP, sind und optional die Schwanzbildung der fragmentierten Nukleinsäuremoleküle in Schritt (ii) G-Schwanzbildung umfasst; oder

(iv) die in Schritt (ii) eingeführten Didesoxynukleotide Didesoxy-Nicht-T-Nukleotide, wie etwa ddVTP, sind und optional die Schwanzbildung der fragmentierten Nukleinsäuremoleküle in Schritt (ii) T-Schwanzbildung umfasst.

**7.** Verfahren nach einem der Ansprüche 1 bis 6, wobei die Sequenzierungsadapter Duplex-Sequenzierungsadapter sind, die eine Strichcodesequenz umfassen.

**8.** Verfahren nach einem der Ansprüche 1 bis 7, wobei die Nukleinsäurebibliothek für Duplex-Sequenzierung vorgesehen ist.

**9.** Verfahren nach Anspruch 8, wobei die Nukleinsäurebibliothek auf eine Konzentration verdünnt ist, die die Duplex-Abdeckung wie bei Verwendung eines analytischen und empirischen Modells vorhergesagt maximiert, optional wobei die Duplex-Abdeckung zwischen ungefähr 15x und 30x Vollgenomäquivalent liegt und die Nukleinsäurebibliothek auf zwischen ungefähr 0,3 fmol und ungefähr 0,6 fmol verdünnt ist.

**10.** Verfahren nach einem der Ansprüche 1 bis 9, zusätzlich den folgenden Schritt umfassend:

(iv) selektives Anreichern von Nukleinsäuren, die einer oder mehreren interessierenden genomischen Regionen entsprechen, um eine Bibliothek angereicherter Nukleinsäuren zu erzeugen,

optional wobei die eine oder mehreren interessierenden genomischen Regionen eine oder mehrere codierende Sequenzen (CDS) codierender Gene, wie etwa Protein-codierende Sequenzen, sind, und optional wobei das Verfahren zum Erzeugen einer Nukleinsäurebibliothek zur Exomsequenzierung vorgesehen ist und das selektive Anreichern von Nukleinsäuren, die interessierenden genomischen Regionen entsprechen, in Schritt (iv) die Verwendung von Oligonukleotidsonden umfasst, die an die codierenden Sequenzen (CDS) binden.

**11.** Verfahren zum Detektieren von in einzelnen DNA-Molekülen vorhandenen Mutationen, wobei das Verfahren die folgenden Schritte umfasst:

(i) Erzeugen einer Nukleinsäurebibliothek oder einer Bibliothek angereicherter Nukleinsäuren unter Verwendung des Verfahrens nach einem der Ansprüche 1 bis 10;

(ii) Sequenzieren der Nukleinsäurebibliothek oder der Bibliothek angereicherter Nukleinsäuren, um Sequenzierungsdaten zu erzeugen; und

(iii) rechnerisches Analysieren der Sequenzierungsdaten von Schritt (ii) zum Detektieren des Vorhandenseins und/oder der Identität einer Mutation in einzelnen DNA-Molekülen,

optional wobei das rechnerische Analysieren von Schritt (iii) Folgendes umfasst:

(a) Ausrichten an einem Referenzgenom;

(b) Gruppieren von Lesevorgängen der Sequenzierungsdaten unter Verwendung einer Kombination von Fragmentierungsbruchpunktpositionen, Duplex-Strichcodesequenzen, Sequenzierungslesevorgängen und Strangidentitäten oder Identifizieren und Gruppieren von Lesevorgängen der Sequenzierungsdaten basierend auf der Position in dem Referenzgenom, dem sie entsprechen, und/oder dem Nukleinsäurefragment, von dem sie abgeleitet sind;

(c) Bereitstellen einer konsensbasierten Qualitätsbewertung des Basenabrufs; und

(d) Filtern von falsch positiven Basenabrufen sowohl an Referenzpositionen als auch an mutierten Positionen.

12. Verfahren zum Detektieren einer in einem einzelnen DNA-Molekül vorhandenen Mutation aus einer Population von Zellen, umfassend ein Durchführen des Verfahrens nach einem der Ansprüche 1 bis 11,

optional wobei die Population von Zellen eine polyklonale Population von Zellen ist und/oder

optional wobei die Population von Zellen eine gealterte Population von Zellen oder eine kranke Population von Zellen ist und/oder

optional wobei die in einem einzelnen DNA-Molekül vorhandene Mutation eine Treibermutation ist.

13. Verfahren nach Anspruch 12, wobei die Treibermutation an einem Alterungsprozess beteiligt ist oder eine Krankheitstreibermutation ist.

14. Verfahren zum Diagnostizieren einer Krankheit bei einem menschlichen oder tierischen Subjekt, wobei das Verfahren die folgenden Schritte umfasst:

(i) Detektieren des Vorhandenseins und/oder der Identität einer Mutation in einem einzelnen DNA-Molekül gemäß dem Verfahren nach Anspruch 12 oder Anspruch 13; und

(ii) Verwenden des Vorhandenseins und/oder der Identität der Mutation als Indikator für eine Krankheit bei dem menschlichen oder tierischen Subjekt.

15. In-vitro-Verfahren zum Detektieren des Vorhandenseins und/oder der Identität einer vermuteten Mutation in einer Zelle, wobei das Verfahren die folgenden Schritte umfasst:

(i) Kultivieren einer Zelle *in vitro* und/oder Unterziehen einer In-vitro-Zellkultur einem mutagenen Prozess, wie etwa durch Durchführen von Geneditierung; und

(ii) Detektieren des Vorhandenseins und/oder der Identität einer Mutation in einem einzelnen DNA-Molekül in der Zelle oder Zellkultur gemäß dem Verfahren nach Anspruch 12 oder Anspruch 13.

**Revendications**

1. Procédé de génération d'une bibliothèque d'acides nucléiques pour un séquençage, ledit procédé comprenant les étapes de :

(i) fragmentation d'une composition d'acide nucléique pour générer des molécules d'acide nucléique fragmentées avec des extrémités franches ;

(ii) introduction de didésoxynucléotides au niveau de sites d'entaille interne présents dans les molécules d'acide nucléique fragmentées avec des extrémités franches et d'une queue pour générer des fragments d'acide nucléique à queue ; et

(iii) ajout d'adaptateurs de séquençage aux fragments d'acide nucléique à queue pour générer une bibliothèque d'acides nucléiques.

2. Procédé selon la revendication 1, dans lequel la fragmentation de la composition d'acide nucléique à l'étape (i) comprend l'utilisation d'une ou plusieurs endonucléases génératrices d'extrémités franches/non cohésives,

éventuellement dans lequel l'utilisation d'une endonucléase génératrice d'extrémités franches/non cohésives évite la nécessité d'une réparation d'extrémités des molécules d'acide nucléique fragmentées, et/ou

éventuellement dans lequel l'endonucléase génératrice d'extrémités franches/non cohésives est une endonucléase de restriction génératrice d'extrémités franches/non cohésives ayant un site de reconnaissance de 4 paires de bases et/ou qui n'est pas altérée par une méthylation CpG chevauchante,

éventuellement dans lequel l'endonucléase de restriction génératrice d'extrémités franches/non cohésives est choisie parmi l'un ou les deux parmi : AluI et HpyCH4, tels que HpyCH4III, HpyCH4IV ou HpyCH4V.

3. Procédé selon la revendication 1, dans lequel la fragmentation de la composition d'acide nucléique à l'étape (i) comprend l'élimination de surplombs des molécules d'acide nucléique fragmentées à l'aide d'une ou plusieurs enzymes exonucléases pour générer des molécules d'acide nucléique fragmentées avec des extrémités franches,

éventuellement dans lequel la fragmentation de la composition d'acide nucléique à l'étape (i) comprend une fragmentation mécanique, telle que la sonication.

4. Procédé de génération d'une bibliothèque d'acides nucléiques pour un séquençage, ledit procédé comprenant les étapes de :

(ia) fragmentation d'une composition d'acide nucléique par sonication pour générer des molécules d'acide nucléique fragmentées ;
(ib) élimination de surplombs des molécules d'acide nucléique fragmentées à l'aide d'une ou plusieurs enzymes exonucléases pour générer des molécules d'acide nucléique fragmentées avec des extrémités franches;
(ii) introduction de didésoxynucléotides au niveau de sites d'entaille interne présents dans les molécules d'acide nucléique fragmentées et d'une queue pour générer des fragments d'acide nucléique à queue ; et
(iii) ajout d'adaptateurs de séquençage aux fragments d'acide nucléique à queue pour générer une bibliothèque d'acides nucléiques.

5. Procédé selon la revendication 3 ou la revendication 4, dans lequel l'une ou les plusieurs enzymes exonucléases sont la nucléase de haricot mungo.

6. Procédé selon l'une quelconque des revendications 1 à 5, dans lequel l'introduction de didésoxynucléotides au niveau de sites d'entaille + à l'étape (ii) empêche l'extension de l'entaille interne et conduit à un brin d'acide nucléique non amplifiable, et/ou
dans lequel :

(i) les didésoxynucléotides introduits à l'étape (ii) sont des didésoxynucléotides non-A, tels que des ddBTP, et éventuellement la queue des molécules d'acide nucléique fragmentées à l'étape
(ii) comprend une queue A ; ou
(ii) les didésoxynucléotides introduits à l'étape (ii) sont des didésoxynucléotides non-C, tels que des ddDTP, et éventuellement la queue des molécules d'acide nucléique fragmentées à l'étape (ii) comprend une queue C ; ou
(iii) les didésoxynucléotides introduits à l'étape (ii) sont des didésoxynucléotides non-G, tels que des ddHTP, et éventuellement la queue des molécules d'acide nucléique fragmentées à l'étape (ii) comprend une queue G ; ou
(iv) les didésoxynucléotides introduits à l'étape (ii) sont des didésoxynucléotides non-T, tels que des ddVTP, et éventuellement la queue des molécules d'acide nucléique fragmentées à l'étape (ii) comprend une queue T.

7. Procédé selon l'une quelconque des revendications 1 à 6, dans lequel les adaptateurs de séquençage sont des adaptateurs de séquençage duplex comprenant une séquence de code-barres.

8. Procédé selon l'une quelconque des revendications 1 à 7, dans lequel la bibliothèque d'acides nucléiques est destinée au séquençage duplex.

9. Procédé selon la revendication 8, dans lequel la bibliothèque d'acides nucléiques est diluée à une concentration qui maximise la couverture duplex telle que prédite à l'aide d'un modèle analytique et empirique,
éventuellement dans lequel la couverture duplex est comprise entre environ 15x et 30x l'équivalent du génome entier et la bibliothèque d'acides nucléiques est diluée entre environ 0,3 fmol et environ 0,6 fmol.

10. Procédé selon l'une quelconque des revendications 1 à 9, comprenant également l'étape de :

(iv) enrichissement sélectif d'acides nucléiques correspondant à une ou plusieurs régions génomiques d'intérêt pour générer une bibliothèque d'acides nucléiques enrichie,
éventuellement dans lequel l'une ou les plusieurs régions génomiques d'intérêt sont une ou plusieurs séquences codantes (CDS) de gènes codants, telles que des séquences codantes de protéines, et éventuellement dans lequel le procédé est destiné à générer une bibliothèque d'acides nucléiques pour le séquençage de l'exome et à enrichir sélectivement des acides nucléiques correspondant aux régions génomiques d'intérêt dans l'étape (iv) comprend l'utilisation de sondes oligonucléotidiques qui se lient aux séquences codantes (CDS).

11. Procédé de détection de mutations présentes dans des molécules d'ADN individuelles, ledit procédé comprenant les étapes de :

(i) génération d'une bibliothèque d'acides nucléiques ou d'une bibliothèque d'acides nucléiques enrichie en utilisant le procédé selon l'une quelconque des revendications 1 à 10 ;
(ii) séquençage de la bibliothèque d'acides nucléiques ou de la bibliothèque d'acides nucléiques enrichie pour générer des données de séquençage ; et
(iii) analyse informatique des données de séquençage de l'étape (ii) pour détecter la présence et/ou l'identité d'une mutation dans des molécules d'ADN individuelles,

éventuellement dans lequel l'analyse informatique de l'étape (iii) comprend :

(a) l'alignement sur un génome de référence ;
(b) le regroupement de lectures des données de séquençage en utilisant une combinaison de positions de points d'arrêt de fragmentation, de séquences de codes-barres duplex, de lecture de séquençage et d'identité de brin, ou l'identification et le regroupement de lectures des données de séquençage sur la base de la position dans le génome de référence à laquelle elles correspondent et/ou du fragment d'acide nucléique dont elles dérivent ;
(c) la fourniture d'un score de qualité d'appel de base consensuel ; et
(d) le filtrage des appels de base faussement positifs aux positions de référence et mutées.

**12.** Procédé de détection d'une mutation présente dans une molécule d'ADN individuelle à partir d'une population de cellules, comprenant la mise en œuvre du procédé selon l'une quelconque des revendications 1 à 11,

éventuellement dans lequel la population de cellules est une population de cellules polyclonales, et/ou
éventuellement dans lequel la population de cellules est une population de cellules âgées ou une population de cellules malades, et/ou
éventuellement dans lequel la mutation présente dans une molécule d'ADN individuelle est une mutation motrice.

**13.** Procédé selon la revendication 12, dans lequel la mutation motrice est impliquée dans un processus de vieillissement ou est une mutation motrice de maladie.

**14.** Procédé de diagnostic d'une maladie chez un sujet humain ou animal, ledit procédé comprenant les étapes de :

(i) détection de la présence et/ou de l'identité d'une mutation dans une molécule d'ADN individuelle selon le procédé de la revendication 12 ou de la revendication 13 ; et
(ii) utilisation de la présence et/ou de l'identité de la mutation comme indicateur de maladie chez le sujet humain ou animal.

**15.** Procédé *in vitro* de détection de la présence et/ou de l'identité d'une mutation suspectée dans une cellule, ledit procédé comprenant les étapes de :

(i) culture d'une cellule *in vitro* et/ou soumission d'une culture cellulaire *in vitro* à un processus mutagène, par exemple en effectuant une édition génétique ; et
(ii) détection de la présence et/ou de l'identité d'une mutation dans une molécule d'ADN individuelle dans la cellule ou dans une culture cellulaire, selon le procédé de la revendication 12 ou de la revendication 13.

FIGURE 1

**FIGURE 1 (continued)**

**FIGURE 1 (continued)**

FIGURE 2

FIGURE 2 (continued)

FIGURE 3

**b**

**c**

**FIGURE 3 (continued)**

**NanoSeq simulation**

FIGURE 4

FIGURE 5

**a**

FIGURE 6

# Colonic crypts - NanoSeq

PD37449

PD34200

PD34201

**FIGURE 6 (continued)**

EP 4 251 763 B1

PD40840

PD47737

FIGURE 6 (continued)

FIGURE 7

FIGURE 7 (continued)

**k**

Healthy donors    Alzheimer's disease donors

Signature contribution

■SigA ▨SigB ▦SigC

**l**  Neurons

Subs / Mb (cohort)

All
T>C
C>T
C>G
T>G
C>A
T>A

Q1 Q2 Q3 Q4
Expression (increasing)

**m**

Indels / bp (cohort)

> 1 bp del
Ins
1 bp del

Q1 Q2 Q3 Q4
Expression (increasing)

## FIGURE 7 (continued)

**FIGURE 7 (continued)**

FIGURE 8

**Mutation rate for each trinucleotide substitution**

FIGURE 9

EP 4 251 763 B1

FIGURE 10

FIGURE 10 (continued)

FIGURE 10 (continued)

**FIGURE 11**

FIGURE 12

**FIGURE 12 (continued)**

FIGURE 12 (continued)

**REFERENCES CITED IN THE DESCRIPTION**

**Patent documents cited in the description**

- WO 2019126803 A
- US 2016215331 A
- WO 2007087310 A